(19) Europäisches Patentamt
European Patent Office
Office européen des brevets

(11) EP 1 159 455 B1

(12) EUROPEAN PATENT SPECIFICATION

(45) Date of publication and mention of the grant of the patent: **26.10.2005 Bulletin 2005/43**

(21) Application number: **00912865.3**

(22) Date of filing: **28.02.2000**

(51) Int Cl.[7]: **C12Q 1/68**, A61K 48/00, A61P 35/00, C12N 9/06

(86) International application number: **PCT/IB2000/000442**

(87) International publication number: **WO 2000/052205 (08.09.2000 Gazette 2000/36)**

# (54) cDNA FOR HUMAN METHYLENETETRAHYDROFOLATE REDUCTASE

CDNA VON MENSCHLICHER METHYLENTETRAHYDROFOLATREDUKTASE

ADNC DESTINES A LA METHYLENE-TETRAHYDROFOLATE REDUCTASE

(84) Designated Contracting States:
**AT BE CH CY DE DK ES FI FR GB GR IE IT LI LU MC NL PT SE**

(30) Priority: **01.03.1999 US 258928**

(43) Date of publication of application: **05.12.2001 Bulletin 2001/49**

(73) Proprietor: **McGill University**
**Montreal, Québec H3A 2T5 (CA)**

(72) Inventors:
- **ROZEN, Rima**
  **Montreal West, Quebec H4X 1R5 (CA)**
- **GOYETTE, Philippe**
  **Montreal, Quebec H4A 2X1 (CA)**

(74) Representative: **Bösl, Raphael Konrad et al**
**Patentanwälte**
**Isenbruck Bösl Hörschler Wichmann Huhn**
**Postfach 860 880**
**81635 München (DE)**

(56) References cited:
**WO-A-95/33054**

- **FROSST P ET AL: "A CANDIDATE GENETIC RISK FACTOR FOR VASCULAR DISEASE: A COMMON MUTATION IN METHYLENETETRAHYDROFOLATE REDUCTASE" NATURE GENETICS,US,NEW YORK, NY, vol. 10, no. 1, 1 May 1995 (1995-05-01), pages 111-113, XP000198175 ISSN: 1061-4036**
- **GOYETTE P ET AL: "SEVERE AND MILD MUTATIONS IN CIS FOR THE METHYLENETETRAHYDROFOLATE RUDUCTASE (MTHFR) GENE, AND DESCRIPTION OF FIVE NOVEL MUTATIONS IN MTHFR" AMERICAN JOURNAL OF HUMAN GENETICS,US,UNIVERSITY OF CHICAGO PRESS, CHICAGO,, vol. 59, no. 6, 1 December 1996 (1996-12-01), pages 1268-1275, XP000672651 ISSN: 0002-9297**
- **SCHWARTZ ET AL: "MYOCARDIAL INFARCTION IN YOUNG WOMEN IN RELATION TO PLASMA TOTAL HOMOCYSTEINE, FOLATE, AND A COMMON VARIANT IN THE METHYLENTETRAHYDROFOLATE REDUCTASE GENE" CIRCULATION,US,AMERICAN HEART ASSOCIATION, DALLAS, TX, vol. 96, no. 2, 1 July 1997 (1997-07-01), pages 412-417, XP002091720 ISSN: 0009-7322**
- **CHAPMAN J ET AL: "ACE, MTHFR, FACTOR V LEIDEN, AND APOE POLYMORPHISMS IN PATIENTS WITH VASCULAR AND ALZHEIMER'S DEMENTIA" STROKE,US,AMERICAN HEART ASSOCIATION, DALLAS TX, vol. 29, no. 7, July 1998 (1998-07), pages 1401-1404, XP000857898 ISSN: 0039-2499**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 159 455 B1

## Description

## BACKGROUND OF THE INVENTION

(a) Field of the Invention

The invention relates to a cDNA probe for human methylenetetrahydrofolate reductase (MTHFR), and its uses.

(b) Description of Prior Art

Folic acid derivatives are coenzymes for several critical single-carbon transfer reactions, including reactions in the biosynthesis of purines, thymidylate and methionine. Methylenetetrahydrofolate reductase (MTHFR; EC 1.5.1.20) catalyses the NADPH-linked reduction of 5,10-methylenetetrahydrofolate to 5-methyltetrahydrofolate, a co-substrate for methylation of homocysteine to methionine. The porcine liver enzyme, a flavoprotein, has been purified to homogeneity; it is a homodimer of 77-kDa subunits. Partial proteolysis of the porcine peptide has revealed two spatially distinct domains: an N-terminal domain of 40 kDa and a C-terminal domain of 37 kDa. The latter domain contains the binding site for the allosteric regulator S-adenosylmethionine.

Hereditary deficiency of MTHFR, an autosomal recessive disorder, is the most common inborn error of folic acid metabolism. A block in the production of methyltetrahydrofolate leads to elevated homocysteine with low to normal levels of methionine. Patients with severe deficiencies of MTHFR (0 -20% activity in fibroblasts) can have variable phenotypes. Developmental delay, mental retardation, motor and gait abnormalities, peripheral neuropathy, seizures and psychiatric disturbances have been reported in this group, although at least one patient with severe MTHFR deficiency was asymptomatic. Pathologic changes in the severe form include the vascular changes that have been found in other conditions with elevated homocysteine, as well as reduced neurotransmitter and methionine levels in the CNS. A milder deficiency of MTHFR (35-50% activity) has been described in patients with coronary artery disease (see below). Genetic heterogeneity is likely, considering the diverse clinical features, the variable levels of enzyme activity, and the differential heat inactivation profiles of the reductase in patients' cells.

Coronary artery disease (CAD) accounts for 25% of deaths of Canadians. Cardiovascular risk factors (male sex, family history, smoking, hypertension, dyslipoproteinemia and diabetes) account for approximately 60 to 70% of the ability to discriminate CAD patients from healthy subjects. Elevated plasma homocysteine has also been shown to be an independent risk factor for cardiovascular disease.

Homocysteine is a sulfhydryl-containing amino acid that is formed by the demethylation of methionine. It is normally metabolized to cysteine (transsulfuration) or re-methylated to methionine. Inborn errors of metabolism (as in severe MTHFR deficiency) causing extreme elevations of homocysteine in plasma, with homocystinuria, are associated with premature vascular disease and widespread arterial and venous thrombotic phenomena. Milder elevations of plasma homocysteine (as in mild MTHFR deficiency) have been associated with the development of peripheral vascular disease, cerebrovascular disease and premature CAD.

Homocysteine remethylation to methionine requires the folic acid intermediate, 5-methyltetrahydrofolate, which is produced from 5,10-methylenetetrahydrofolate folate through the action of 5,10-methylenetetrahydrofolate reductase (MTHFR). Deficiency of MTHFR results in an inability to metabolize homocysteine to methionine; elevated plasma homocysteine and decreased methionine are the metabolic consequences of the block. Severe deficiencies of MTHFR (less than 20% of activity of controls) as described above, are associated with early-onset neurologic symptoms (mental retardation, peripheral neuropathy, seizures, etc.) and with atherosclerotic changes and thromboembolism. Milder deficiencies of MTHFR (35-50% of activity of controls), with a thermolabile form of the enzyme, are seen in patients with cardiovascular disease without obvious neurologic abnormalities.

In a survey of 212 patients with proven coronary artery disease, the thermolabile form of MTHFR was found in 17% of the CAD group and 5% of controls. In a subsequent report on 339 subjects who underwent coronary angiography, a correlation was found between thermolabile MTHFR and the degree of coronary artery stenosis. Again, traditional risk factors (age, sex, smoking, hypertension, etc.) were not significantly associated with thermolabile MTHFR. All the studies on MTHFR were performed by enzymatic assays of MTHFR in lymphocytes, with measurements of activity before and after heat treatment to determine thermolability of the enzyme.

Since 5-methyltetrahydrofolate, the product of the MTHFR reaction, is the primary form of circulatory folate, a deficiency in MTHFR might lead to other types of disorders. For example, periconceptual folate administration to women reduces the occurrence and recurrence of neural tube defects in their offspring. Neural tube defects are a group of developmental malformations (meningomyelocele, anencephaly, and encephalocele) that arise due to failure of closure of the neural tube. Elevated levels of plasma homocysteine have been reported in mothers of children with neural tube defects. The elevated plasma homocysteine could be due to a deficiency of MTHFR, as described above for cardiovascular disease.

Neuroblastomas are tumors derived from neural crest cells. Many of these tumors have been reported to have deletions of human chromosome region 1p36, the region of the genome to which MTHFR has been mapped. It is possible that MTHFR deletions/mutations are responsible for or contribute to the formation of this type of tumor. MTHFR abnormalities may also contribution to the formation of other types of tumors, such as colorectal tumors, since high dietary folate has been shown to be inversely associated with risk of colorectal carcinomas.

MTHFR activity is required for homocysteine methylation to methionine. Methionine is necessary for the formation of S-adenosylmethionine, the primary methyl donor for methylation of DNA, proteins, lipids, neurotransmitters, etc. Abnormalities in MTHFR might lead to lower levels of methionine and S-adenosylmethionine, as well as to elevated homocysteine. Disruption of methylation processes could result in a wide variety of conditions, such as neoplasias, developmental anomalies, neurologic disorders, etc.

Although the MTHFR gene in *Escherichia coli* (*metF*) has been isolated and sequenced, molecular studies of the enzyme in higher organisms have been limited without the availability of an eukaryotic cDNA.

It would be highly desirable to be provided with a cDNA probe for human methylenetetrahydrofolate reductase (MTHFR). This probe would be used for identification of sequence abnormalities in individuals with severe or mild MTHFR deficiency, including cardiovascular patients and patients with neurologic symptoms or tumors. The probe would also be used in gene therapy, isolation of the gene, and expression studies to produce the MTHFR protein. The probe would also provide the amino acid sequence of the human MTHFR protein, which would be useful for therapy of MTHFR deficiency by biochemical or pharmacological approaches.

It would be highly desirable to be provided with a molecular description of mutations in methylenetetrahydrofolate reductase deficiency.

WO 95/33054 A1 describes a cDNA probe for human methylenetetrahydrofolate reductase (MTHFR), and certain uses thereof.

Frosst P. et al., Nature Genetics, voL 10, May 1995, pp. 111-113 describe a common mutation in the cDNA coding for MTHFR, which leads to a thermolabile enzyme and may represent a genetic risk factor in vascular disease.

Goyette P. et al., Am. J. Hum. Genet, 59, 1996, pp. 1268-1275 describe several mutations in the cDNA coding for MTHFR, which lead to a thermolabile enzyme or to severe MTHFR deficiency.

Schwartz S.M. et al., Circulation, vol. 96, no. 2, July 15, 1997, pp. 412-417 examined a possible relationship between a common cytosine to thymine polymorphism in the gene for MTHFR and the risk of myocardial infarction in young women.

Chapman J. et al., Stroke, 29, July 1998, pp. 1401-1404 discuss the suitability of several biological markers for vascular dementia, comprising amongst others the T677C polymorphism of MTHFR, which leads to a thermolabile enzyme.

Patients with sequence abnormalities in MTHFR might have different responses to drugs, possibly but not limited to drugs that affect folate metabolism. Therefore, it would be useful to know if these mutations are present before determining the appropriate therapy. The drugs/diseases for which this might be relevant include cancer chemotherapeutic agents, antibiotics, antiepileptic medicition, antiarthritic medication, etc.

## SUMMARY OF THE INVENTION

Disclosed is a cDNA probe for human methylenetetrahydrofolate reductase (MTHFR).

Also disclosed is a molecular description of mutations in methylenetetrahydrofolate reductase deficiency.

Another disclosure is a nucleic acid and amino acid sequence for human methylenetetrahydrofolate reductase.

Also disclosed is a system for synthesis of MTHFR protein *in vitro.*

A further aim of the present invention is to provide technology/protocol for identification of sequence changes in the MTHFR gene.

In accordance with the present invention, there is provided a use of an in-vitro method for the identification of methylenetetrahydrofolate reductase (MTHFR) deficiency in a patient with MTHFR deficiency. The in-vitro method comprises the steps of amplifying a DNA sample obtained from the patient or reverse-transcripting a RNA sample obtained from the patient into a DNA and amplifying the DNA, and analyzing the amplified DNA to determine at least one sequence abnormality with respect to a human MTHFR encoded by a nucleotide sequence as set forth in SEQ ID NO:1 or having an amino acid sequence as set forth in SEQ ID NO:2, the sequence abnormality being indicative of MTHFR deficiency.

The sequence abnormality may comprise a mutation selected from a group consisting of 167G→A, 482G→A, 559C→T, 677C→T, 692C→T, 764C→T, 792+1G→A, 985C→T, 1015C→T, 1081C→T, 1298A→C and 1317T→C.

The selected mutation may consist of 677C→T.

The MTHFR deficiency is associated with osteoporosis.

In accordance with yet another aspect of the present invention, there is provided a use of a recombinant vector for expression of MTHFR under the control of a suitable promoter, the MTHFR being encoded by a nucleotide sequence as set forth in SEQ ID NO:1 or having an amino acid sequence as set forth in SEQ ID NO:2, for the manufacture of a medicament for the treatment of osteoporosis in a patient wherein said osteoporosis is associated with MTHFR deficiency.

In accordance with yet another aspect of the present invention, there is provided a use of a methylenetetrahydrofolate reductase (MTHFR) protein encoded by a nucleotide sequence as set forth in SEQ ID NO. 1 or having an amino acid sequence as set forth in SEQ ID NO.2 for the manufucture of a medicament for the treatment of osteoporosis in a patient wherin said osteoporosis is associated with methylenetetrahydrofolate reductase (MTHFR) deficiency.

MTHFR deficiency may be associated with a disorder selected from a group consisting of cardiovascular disorders, coronary and arterial disorders, neurological disorders, increased risk of occurrence of a neural tube defect in an offspring, cancer, osteoporosis and other disorders influenced by folic acid metabolism.

A "polymorphism" is intended to mean a mutation present in 1% or more of alleles of the general population. A polymorphism is disease-causing when it is present in patients with a disease but not in the general population. However, a polymorphism present both in patients having a disease and in the general population is not necessarily benign. The definition of a disease-causing substitution, as distinct from a benign polymorphism, is based on 3 factors: (1) absence of the change in at least 50 independent control chromosomes; (2) presence of the amino acid in the bacterial enzyme, attesting to its evolutionary significance and (3) change in amino acid not conservative. Although expression of the substitutions is required to formally prove that they are not benign, the criteria above allow us to postulate that the changes described in this report are likely to affect activity.

## BRIEF DESCRIPTION OF THE DRAWINGS

Figs. 1A to 1F illustrate the first cDNA coding sequence (SEQ ID NO:1 and NO:2) for methylenetetrahydrofolate reductase (MTHFR);

Fig. 2 is the alignment of amino acids for human methylenetetrahydrofolate reductase (MTHFR), the *metF* genes from *E. Coli* (ECOMETF), and *S. Typhimurium* (STYMETF), and an unidentified open reading frame in *Saccharomyces cerevisiae* that is divergently transcribed from an excision repair gene (ysRAD1);

Figs. 3A and 3B illustrate the sequencing and restriction enzyme analysis for the Arg to Ter substitution;

Figs. 4A and 4B illustrate the sequencing and restriction enzyme analysis for the Arg to Gln substitution;

Figs. 5A and 5B illustrate the sequence change and restriction enzyme analysis for the alanine to valine substitution;

Figs. 6A to 6C illustrate the total available sequence (SEQ ID NO:3 and NO:4) of human MTHFR cDNA;

Figs. 7A and 7B illustrate the expression analysis of MTHFR cDNA in *E. Coli, respectively (7A)* the Western blot of bacterial extracts and tissues, and (7B) the thermolability assay of bacterial extracts;

Figs. 8A to 8D illustrate the identification of a 5' splice site mutation leading to a 57-bp in-frame deletion of the cDNA;

Figs. 9A to 9D illustrate the diagnostic restriction endonuclease analysis of 4 mutations;

Figs. 10A to 10D illustrate the ASO hybridization analysis of 2 mutations;

Fig. 11 illustrates the region of homology between human methylenetetrahydrofolate reductase (MTHFR) and human dihydrofolate reductase (DHFR);

Figs. 12A-12B illustrate the exonic sequences of the human MTHFR gene with their flanking intronic sequences;

Figs. 13A-13B illustrate the exonic sequences of the mouse MTHFR gene with their flanking intronic sequences;

Fig. 14 illustrates intron sizes and locations for both human and mouse genes; and

Fig. 15 illustrates the alignment of MTHFR amino acid sequences for the human MTHFR (hMTHFR), mouse MTHFR (mMTHFR) and the MetF gene of bacteria (bMTHFR).

## DETAILED DESCRIPTION OF THE INVENTION

### Sequencing of peptides from porcine MTHFR

Homogeneous native porcine MTHFR was digested with trypsin to generate a 40 kDa N-terminal fragment and a 31 kDa C-terminal fragment; the 31 kDa fragment is a proteolytic product of the 37 kDa fragment. The fragments were separated by SDS-PAGE, electroeluted, and the denatured fragments were digested with lysyl endopeptidase (LysC). The resulting peptides were separated by reversed-phase HPLC and subjected to sequence analysis by Edman degradation (details contained in Goyette P et al., *Nature Genetics,* 1994, 7:195-200).

**Isolation and sequencing of cDNAs**

Two degenerate oligonucleotides were synthesized based on the sequence of a 30 amino acid porcine MTHFR peptide (first underlined peptide in Fig. 2). These were used to generate a 90 bp PCR product, encoding the predicted peptide, from reverse transcription-PCR reactions of 500 ng pig liver polyA+ RNA. A pig-specific (non-degenerate, antisense) PCR primer was then synthesized from this short cDNA sequence. Using this primer and a primer for phage arms, a human liver λgt10 cDNA library (Clontech) was screened by PCR; this technique involved the generation of phage lysate stocks (50,000 pfu) which were boiled for 5 min and then used directly in PCR reactions with these two primers. PCR fragments were then sequenced directly (Cycle Sequencing™ kit, GIBCO), and a positive clone was identified by comparison of the deduced amino acid sequence to the sequence of the pig peptide (allowing for inter-species variations). The positive stock was then replated at lower density and screened with the radiolabelled positive PCR product by plaque hybridization until a well-isolated plaque was identified. Phage DNA was purified and the insert was then subcloned into pBS+ (Bluescript) and sequenced on both strands (Cycle Seclluencing™ kit, GIBCO and Sequenase™, Pharmacia). The deduced amino acid sequence of the human cDNA was aligned to the porcine peptide sequences, the *metF* genes from E. coli (ecometf, accession number VO1502) and *S. Typhimurium* (stymetF, accession number XO7689) and with a previously unidentified open reading frame in *Saccharomyces cerevisiae* that is divergently transcribed with respect to the excision repair gene, *ysRAD1* (accession number KO2070). The initial alignments were performed using BestFit™ in the GCG computer package, and these alignments were adjusted manually to maximize homologies.

In summary, degenerate oligonucleotide primers were designed to amplify a sequence corresponding to a 30-amino acid segment of a porcine peptide from the N-terminal region of the enzyme (first porcine peptide in Fig. 2). A 90-bp porcine cDNA fragment was obtained from reverse transcription/PCR of pig liver RNA. Sequencing of the PCR fragment confirmed its identity by comparison of the deduced amino acid sequence to the porcine peptide sequence. A nondegenerate oligonucleotide primer, based on the internal sequence of the porcine cDNA, was used in conjunction with primers for the phage arms to screen a human liver λgt10 cDNA library by PCR. The insert of the positive clone was isolated and sequenced. The sequence consisted of 1266 bp with one continuous open reading frame.

**Homology with MTHFR in other species**

The deduced amino acid sequence of the human cDNA was aligned with the *metF* genes from *E. coli* and *S. typhimurium*, as well as with a previously unidentified ORF in *Saccharomyces cerevisiae* that is divergently transcribed with respect to the excision repair gene, *ysRAD1* (Fig. 2). The sequences homologous to 5 porcine peptides are underlined in Fig. 2. Three segments (residues 61-94, 219-240, and 337-351) correspond to internal peptide sequence from the N-terminal 40-kDa domain of the porcine liver enzyme. Residues 374-393 correspond to the upstream portion of the LysC peptide from the C-terminal domain of the porcine liver enzyme that is labeled when the enzyme is irradiated with UV light in the presence of ($^3$H-methyl)AdoMet; as predicted from the AdoMet labeling studies, this peptide lies at one end (N-terminal) of the 37 kDa domain. A fifth region of homology (residues 359-372) was also identified, but the localization of the porcine peptide within the native protein had not been previously determined.

Methylenetetrahydrofolate reductase (MTHFR) is an enzyme involved in amino acid metabolism, that is critical for maintaining an adequate methionine pool, as well as for ensuring that the homocysteine concentration does not reach toxic levels. The high degree of sequence conservation, from *E. coli* to *Homo sapiens*, attests to the significance of MTHFR in these species. The enzyme in *E. coli* (encoded by the metF locus) is a 33-kDa peptide that binds reduced FAD and catalyzes the reduction of methylenetetrahydrofolate to methyltetrahydrofolate. The *metF* enzyme differs from the mammalian enzyme in that NADPH or NADH cannot reduce it, and its activity is not allosterically regulated by S-adenosylmethionine. The native porcine enzyme is susceptible to tryptic cleavage between the N-terminal 40 kDa domain and the C-terminal 37 kDa domain, and this cleavage results in the loss of allosteric regulation by adenosylmethionine, but does not result in loss of catalytic activity. Since the homology between the bacterial and mammalian enzymes is within the N-terminal domain, this region must contain the flavin binding site and residues necessary to bind the folate substrate and catalyze its reduction. The domain structure of the human enzyme has not been elucidated, although the human enzyme has been reported to have a molecular mass of 150 kDa and is likely to be a homodimer of 77 kDa.

The predicted point of cleavage between the two domains lies between residues 351 and 374 of the human sequence, based on the localization of peptides obtained from the isolated domains of the porcine enzyme. This region, containing the highly charged sequence KRREED, is predicted to have the highest hydrophilicity and surface probability of any region in the deduced human sequence.

The N-terminus of the porcine protein has been sequenced, and the region encoding this part of the protein is missing from the human cDNA. It is estimated that this cDNA is missing only a few residues at the N-terminus, since the predicted molecular mass of the deduced sequence upstream of the putative cleavage site (KRREED) is 40 kDa, and the measured molecular mass of the porcine N-terminal domain is also 40 kDa. When the bacterial, yeast and human sequences are aligned, the deduced human sequence contains an N-terminal extension of 40 amino acids; it is suspected that this extension contains determinants for NADPH binding. Many pyridine nucleotide-dependent oxidoreductases contain such determinants at the N-terminus of the protein.

The C-terminus of the human sequence contains a peptide that is labeled when the protein is irradiated with ultraviolet light in the presence of tritiated AdoMet. The cDNA sequence reported here contains only about 7 kDa of the predicted 37-kDa mass of this domain, indicating that this cDNA is truncated at the 3' terminus as well. A number of peptides from the C-terminal porcine domain have also not been detected. As might be expected, given that the prokaryotic enzymes do not appear to be allosterically regulated by AdoMet, there are no significant homologies between the C-terminal region in this cDNA and the prokaryotic *metF* sequences. The alignment shown in Fig. 2 shows that the homologous sequences terminate just prior to the putative cleavage site of the human enzyme.

**Chromosomal assignment**

In situ hybridization to metaphase human chromosomes was used for localization of the human gene. The analysis of the distribution of 200 silver grains revealed a significant clustering of grain 40 grains, in the p36.3-36.2 region of chromosome 1 ($p<0.0001$), with the majority of grains, 25 grains, observed over 1p36.3.

The isolation of the human cDNA has allowed us to localize the gene to chromosome 1p36.3. The observation of one strong signal on that chromosome with little background is highly suggestive of a single locus with no pseudogenes. Southern blotting of human DNA revealed fragments of approximately 10 kb, predicting a gene of average size, since this cDNA encodes approximately half of the coding sequence.

**Additional cDNA sequences and constructs for expression analysis**

A human colon carcinoma cDNA library (gift of Dr. Nicole Beauchemin, McGill University) was screened by plaque hybridization with the original 1.3-kb cDNA to obtain additional coding sequences. A cDNA of 2.2 kb was isolated, which contained 1.3 kb of overlapping sequence to the original cDNA and 900 additional bp at the 3' end (Fig. 6). The amino acid sequence is identical to that of the original cDNA for the overlapping segment (codons 1-415) except for codon 177 (ASP) which was a GLY codon in the original cDNA. Analysis of 50 control chromosomes revealed an ASP codon at this position. The cDNA has an open reading frame of 1980 bp, 100 bp of 3' UTR and a poly A tail.

Sequencing was performed on both strands for the entire cDNA. Additional 5' sequences (800 bp) were obtained from a human kidney cDNA library (Clontech) but these sequences did not contain additional coding sequences and were therefore used for the PCR-based mutagenesis only -(as described below) and not for the expression analysis. The two cDNAs (2.2 kb and 800 bp) were ligated using the *EcoR*I site at bp 199 and inserted into the Bluescript™ vector (Stratagene). The 2.2 kb cDNA was subcloned into the expression vector pTrc99A (Pharmacia) using the *Nco*I site at bp 11 and the *Xba*I site in the polylinker region of both the Bluescript™ and the pTrc99A vectors. Sequencing was performed across the cloning sites to verify the wild-type construct.

## UTILITY OF INVENTION IN IDENTIFICATION OF MUTATIONS

### I. Identification of first two mutations in severe MTHFR deficiency

Total RNA of skin fibroblasts from MTHFR-deficient patients was reverse-transcribed and amplified by PCR for analysis by the single strand conformation polymorphism (SSCP) method (Orita, M. et al., Genomics, 1989, 5: 8874-8879). Primers were designed to generate fragments of 250-300 bp and to cover the available cDNA sequences with small regions of overlap for each fragment at both ends. The first mutation identified by SSCP was a C to T substitution at bp 559 in patient 1554; this substitution converted an arginine codon to a termination codon (Fig. 3A). Since the mutation abolished a *FokI* site, restriction digestion was used for confirmation of the change and for screening additional patients for this mutation; a second patient (1627) was identified in this manner (Fig. 3B). The SSCP pattern for patient 1554 and the restriction digestion pattern for both patients was consistent with a homozygous mutant state or with a genetic compound consisting of the nonsense mutation with a second mutation that did not produce any detectable RNA (null allele). Studies in the parents are required for confirmation.

The second substitution (Fig. 4A) was a G to A transition at bp 482 in patient 1834 that converted an arginine into a glutamine residue. The substitution created a *PstI* site that was used to verify the substitution and to identify a second patient (1863) with this change (Fig. 4B). The SSCP analysis and the restriction digestion pattern were consistent with a heterozygous state for both patients. The arginine codon affected by this change is an evolutionarily conserved residue, as shown in Fig. 2. This observation, in conjunction with the fact that the codon change is not conservative, makes a strong argument that the substitution is a pathologic change rather than a benign polymorphism.

Furthermore, 35 controls (of similar ethnic background to that of the probands) were tested for this substitution by Southern blotting of *PstI*-digested DNA; all were negative.

The family of patient 1834 was studied. The symptomatic brother and the mother of the proband were all shown to carry this substitution, whereas the father was negative for the change (Fig. 4B). In the family of 1863, the mother of the proband was shown to be a carrier, while the father and an unaffected brother were negative.

**Cell lines**

Cell line 1554 is from a Hopi male who was admitted at age three months with homocystinuria, seizures, dehydration, corneal clouding, hypotonia and *Candida sepsis.* Folate distribution in cultured fibroblasts showed a *Pediococcus cerivisiae*/*Lactobacillus* casei (PC/LC) ratio of 0.52 (Control 0.14). There was no measurable methylenetetrahydrofolate reductase (MTHFR) activity (Control values = 9.7 and 15.1 nmoles/h/mg protein; residual activity after treatment of control extracts at 55°C for 20 min. = 28% and 31%).

Cell line 1627 is from a Choctaw male who presented with poor feeding, apnea, failure to thrive, dehydration and homocystinuria at five weeks of age. He was subsequently found to have superior sagittal sinus thrombosis and hydrocephalus. The PC/LC ratio was 0.61 and the specific activity of MTHFR was 0.1 nmoles/h/mg protein. There is consanguinity in that the maternal and paternal grandmothers are thought to be "distantly related".

Cell line 1779 is from a French Canadian male with homocystinuria who first had limb weakness, uncoordination, paresthesiae, and memory lapses at age 15 years, and was wheelchair-bound in his early twenties. His brother (cell line 1834) also has homocystinuria, but is 37 years old and asymptomatic. Specific activity of MTHFR was 0.7 and 0.9 nmole/h/mg protein for 1779 and 1834, respectively; the residual activity after heat treatment at 55°C was 0.9% and 0% for 1779 and 1834, respectively.

Cell line 1863 is from a white male who was diagnosed at age 21 years because of a progressive gait disturbance, spasticity, cerebral white matter degeneration, and homocystinuria. He had a brother who died at age 21 years of neurodegenerative disease. Specific activity of MTHFR in fibroblast extracts was 1.76 nmoles/h/mg protein and the residual enzyme activity after treatment at 55°C was 3.6%.

**Mutation analysis**

Primers were designed from the cDNA sequence to generate 250-300 bp fragments that overlapped 50-75 bp at each end. The primer pairs were used in reverse transcription-PCR of 5μg patient total fibroblast RNA. The PCR products were analyzed by a non-isotopic rapid SSCP protocol (PhastSystem™, Pharmacia), which uses direct silver staining for detection of single strands. Any PCR products from patients showing a shift on SSCP gels were purified by NuSieve (FMC Bioproducts) and sequenced directly (Cycle Sequencing™ kit, GIBCO) to identify the change. If the change affected a restriction site, then a PCR product was digested with the appropriate restriction endonuclease and analyzed on polyacrylamide gels. To screen for the Arg to Gln mutation in controls, 5 μg of *PstI*-digested DNA was run on 0.8% agarose gels and analyzed by Southern blotting using the radiolabelled cDNA by standard techniques.

**II. Seven additional mutations at the methylenetetrahydrofolate reductase (MTHFR) locus with genotype: phenotype correlation in severe MTHFR deficiency**

It is reported hereinbelow the characterization of 7 additional mutations at this locus: 6 missense mutations and a 5' splice site defect which activates a cryptic splice site in the coding sequence. A preliminary analysis of the relationship between genotype and phenotype for all 9 mutations identified thus far at this locus is also reported. A nonsense mutation and 2 missense mutations (proline to leucine and threonine to methionine) in the homozygous state are associated with extremely low activity (0-3%) and onset of symptoms within the first year. Other missense mutations (arginine to cysteine and arginine to glutamine) are associated with higher enzyme activity and later onset of symptoms.

7 additional mutations at the MTHFR locus are described and the association between genotype, enzyme activity, and clinical phenotype in severe MTHFR deficiency is examined.

**Patient description**

The clinical and laboratory findings of the patients have been reported in the published literature. Residual MTHFR activity was previously measured in cultured fibroblasts at confluence.

Patient 354, an African-American girl, was diagnosed at age 13 years with mild mental retardation. Her sister, patient 355 was diagnosed at age 15 years with anorexia, tremor, hallucinations and progressive withdrawal. In patient 354, residual MTHFR activity was 19% and in her sister, 355, it was 14% of control values. The residual activity after heating had equivalent thermal stability to control enzyme.

Patient 1807, a Japanese girl whose parents are first cousins, had delayed walking and speech until age 2 years, seizures at age 6 years and a gait disturbance with peripheral neuropathy at age 16 years. Residual activity of MTHFR was 3% and the enzyme was thermolabile.

Patient 735, an African-Indian girl, was diagnosed at age 7 months with microcephaly, progressive deterioration of mental development, apnea and coma. Residual activity of MTHFR was 2% of control levels. Thermal properties were not determined.

Patient 1084, a Caucasian male, was diagnosed at age 3 months with an infantile fibrosarcoma. He was found to be hypotonic and became apneic. He died at the age of 4 months. Residual activity of MTHFR was not detectable. Thermal properties were not determined.

Patient 356, the first patient reported with MTHFR deficiency, is an Italian-American male who presented at age 16 years with muscle weakness, abnormal gait and flinging movements of the upper extremities. MTHFR residual activity was 20% of control values; activity was rapidly and exponentially inactivated at 55°.

Patient 458, a Caucasian male, was diagnosed at age 12 years with ataxia and marginal school performance. Residual MTHFR activity was approximately 10%, and the activity was thermolabile.

Patient 1396, a Caucasian female, was described as clumsy and as having a global learning disorder in childhood. At age 14 years, she developed ataxia, foot drop, and inability to walk. She developed deep vein thrombosis and bilateral pulmonary emboli. Residual activity of MTHFR was 14% and the enzyme was thermolabile.

**Genomic structure and intronic primers**

Exon nomenclature is based on available cDNA sequence in Goyette et al. (Nature *Genetics,* 1994, 7: 195-200). Exon 1 has been arbitrarily designated as the region of cDNA from bp 1 to the first intron. Identification of introns was performed by amplification of genomic DNA using cDNA primer sequences. PCR products that were greater in size than expected cDNA sizes were sequenced directly.

**Mutation detection**

Specific exons (see Table 1 for primer sequences) were amplified by PCR from genomic DNA and analyzed by the SSCP protocol. SSCP was performed with the Phastgel™ system (Pharmacia), a non-isotopic rapid SSCP protocol, as previously described (Goyette P et al., Nature Genetics, 1994, 7:195-200), or with $^{35}$S-labeled PCR products run on 6% acrylamide: 10% glycerol gels at room temperature (6 watts, overnight). In some cases, the use of restriction endonucleases, to cleave the PCR product before SSCP analysis, enhanced the detection of band shifts. PCR fragments with altered mobility were sequenced directly (GIBCO, Cycle Sequencing™ kit). If the sequence change affected a restriction endonuclease site, then the PCR product was digested with the appropriate enzyme and analyzed by PAGE. Otherwise, allele-specific oligonucleotide (ASO) hybridization was performed on a dot blot of the PCR-amplified exon.

**Table 1**

**PCR Primers for DNA amplification and mutation analysis of MTHFR**

| Exon | Primer Type | Primer Sequence (5'→3') | Location | Fragment Size (bp) |
|---|---|---|---|---|
| 1 | Sense | AGCCTCAACCCCTGCTTGGAGG (SEQ ID NO:5) | C | 271 |
| | Antisense | TGACAGTTTGCTCCCCAGGCAC (SEQ ID NO:6) | I | |
| 4 | Sense | TGAAGGAGAAGGTGTCTGCGGGA (SEQ ID NO:7) | C | 198 |
| | Antisense | AGGACGGTGCGGTGAGAGTGG (SEQ ID NO:8) | I | |
| 5 | Sense | CACTGTGGTTGGCATGGATGATG (SEQ ID NO:9) | I | 392 |
| | Antisense | GGCTGCTCTTGGACCCTCCTC (SEQ ID NO:10) | I | |
| 6 | Sense | TGCTTCCGGCTCCCTCTAGCC (SEQ ID NO:11) | I | 251 |
| | Antisense | CCTCCCGCTCCCAAGAACAAAG (SEQ ID NO:12) | I | |

## Table 2

### Summary of genotypes, enzyme activity, age at onset, and background of patients with MTHFR deficiency

| Patient [a] | BPChanges[b] | Amino acid changes | % Activity | Age at Onset | Background |
|---|---|---|---|---|---|
| 1807 | C764T/C764T | Pro→Leu/Pro→Leu | 3 | within 1st year | Japanese |
| 735 | C692T/C692T | Thr→Met/Thr→Met | 2 | 7 months | African Indian |
| 1084 | C692T/C692T | Thr→Met/Thr→Met | 0 | 3 months | Caucasian |
| 1554 | C559T/C559T | Arg→Ter/Arg→Ter | 0 | 1 month | Native American (Hopi) |
| 1627 | C559T/C559T | Arg→Ter/Arg→Ter | 1 | 1 month | Native American (Choctaw) |
| 356 | C985T/C985T | Arg→Cys/Arg→Cys | 20 | 16 yrs | Italian American |
| 458 | C1015T/G167A | Arg→Cys/Arg→Gln | 10 | 11 yrs | Caucasian |
| 1396 | C1081T/G167A | Arg→Cys/Arg→Gln | 14 | 14 yrs | Caucasian |
| 1779[c] | G482A/? | Arg→Gln/? | 6 | 15 yrs | French Canadian |
| 1834[c] | G482A/? | Arg→Gln/? | 7 | Asymptomatic at 37 yrs | French Canadian |
| 1863 | G482A/? | Arg→Gln/? | 14 | 21 yrs | Caucasian |
| 354[d] | 792 + 1G→A/? | 5' splice site/? | 19 | 13 yrs | African American |
| 355[d] | 792 + 1G→A/? | 5' splice site/? | 14 | 11 yrs | African American |

a Patients 1554, 1627, 1779, 1834 and 1863 were previously reported by Goyette et al. (1994).
b ? = unidentified mutation.
c Patients 1779 and 1834 are sibs.
d Patients 354 and 355 are sibs.

(1) 5' splice site mutation

Amplification of cDNA, bp 653-939, from reverse-transcribed total fibroblast RNA revealed 2 bands in sisters 354 and 355: a smaller PCR fragment (230 bp) in addition to the normal 287 bp allele (Fig. 8A). Fig. 8A is the PAGE analysis of amplification products of cDNA bp 653-939, from reverse transcribed RNA. Controls have the expected 287-bp fragment while patients 354 and 355 have an additional 230-bp fragment. Sequencing of the smaller fragment identified a 57-bp in-frame deletion which would remove 19 amino acids (Fig. 8B). Fig. 8B is the direct sequencing of the PCR products from patient 354. The 57-bp deletion spans bp 736-792 of the cDNA. An almost perfect 5' splice site (boxed) is seen at the 5' deletion breakpoint. Analysis of the sequence at the 5' deletion breakpoint in the undeleted fragment revealed an almost perfect 5' splice site consensus sequence (AG/gcatgc). This observation suggested the presence of a splicing mutation in the natural 5' splice site that might activate this cryptic site, to generate the deleted allele. The sequence following the deletion breakpoint, in the mutant allele, corresponded exactly to the sequence of the next exon. Amplification of genomic DNA, using the same amplification primers as those used for reverse-transcribed RNA, generated a 1.2-kb PCR product indicating the presence of an intron. Direct sequencing of this PCR fragment in patient 354 identified a heterozygous G→A substitution in the conserved GT dinucleotide of the intron at the 5' splice site (Fig. 8C). Fig. 8C is the sequencing of the 5' splice site in control and patient 354. The patient carries a heterozygous G→A substitution in the 5' splice site (boxed). Intronic sequences are in lower case. This substitution abolished a HphI restriction endonuclease site which was used to confirm the mutation in the 2 sisters (Fig. 8D). Fig. 8D is the *Hph*I restriction endonuclease analysis on PCR products of DNA for exon 4 of patients 354 and 355, and of 3 controls (C). The 198-bp PCR product has 2 HphI sites. The products of digestion for the control allele are 151, 24 and 23 bp. The products of digestion for the mutant allele are 175 and 23 bp due to the loss of a HphI site. The fragments of 24 and 23 bp have been run off the gel.

(2) Patients with homozygous coding substitutions

SSCP analysis of exon 4 for patient 1807 revealed an abnormally-migrating fragment, which was directly sequenced to reveal a homozygous C→T substitution (bp 764) converting a proline to a leucine residue. This change creates a *Mnl*I restriction endonuclease site, which was used to confirm the homozygous state of the mutation (Fig. 9A). Fig. 9A is the *Mnl*I restriction analysis of exon 4 PCR products for patient 1807 and 3 controls (C). Expected fragments: control allele, 90, 46, 44, 18 bp; mutant allele, 73, 46, 44, 18, 17 bp. An additional band at the bottom of the gel is the primer. Fifty independent control Caucasian chromosomes and 12 control Japanese chromosomes were tested by restriction analysis; all were negative for this mutation. Homozygosity in this patient is probably due to the consanguinity of the parents.

Patients 735 and 1084 had the same mutation in exon 4, in a homozygous state: a C→T substitution (bp 692) which converted an evolutionarily conserved threonine residue to a methionine residue, and abolished a *Nla*III restriction endonuclease site. Allele-specific oligonucleotide hybridization to amplified exon 4 (Figs. 10A and 10B) was used to confirm the mutation in these 2 patients and to screen 60 independent chromosomes, all of which turned out to be negative. Fig. 10A is the hybridization of mutant oligonucleotide (692T) to exon 4 PCR products from patients 735, 1084 and 30 controls. Only DNA from patients 735 and 1084 hybridized to this probe. Fig. 10B is the hybridization of normal oligonucleotide (692C) to stripped dot blot from A. All control DNAs hybridized to this probe.

Patient 356 showed a shift on SSCP analysis of exon 5. Direct sequencing revealed a homozygous C→T substitution (bp 985) which converted an evolutionarily conserved arginine residue to cysteine; the substitution abolished an AciI restriction endonuclease site. This was used to confirm the homozygous state of the mutation in patient 356 (Fig. 9B) and its presence in the heterozygous state in both parents. Fifty independent control chromosomes, tested in the same manner, were negative for this mutation. Fig. 9B is the *Aci*I restriction analysis of exon 5 PCR products for patient 356, his father (F), his mother (M), and 3 controls (C). Expected fragments: control allele, 129, 105, 90, 68 bp; mutant allele, 195, 129, 68 bp.

(3) Patients who are genetic compounds

Patient 458 is a compound heterozygote of a mutation in exon 5 and a mutation in exon 1. The exon 5 substitution (C→T at bp 1015) resulted in the substitution of a cysteine residue for an arginine residue; this abolished a *Hha*I restriction endonuclease site, which was used to confirm the mutation in patient 458 (Fig. 9C) and to show that 50 control chromosomes were negative. Fig. 9C is the *Hha*I restriction analysis of exon 5 PCR products for patient 458 and 4 controls (C). Expected fragments: control allele, 317 and 75 bp; mutant allele 392 bp. The 75-bp fragment is not shown in Fig. 9C. The second mutation was a heterozygous G→A substitution (bp 167) converting an arginine to a glutamine residue. Allele-specific oligonucleotide hybridization to amplified exon 1 confirmed the heterozygous state of this mutation in patient 458 and identified a second patient (1396) carrying this mutation also in the heterozygous state (Figs. 10C and 10D). Fig. 10C is the hybridization of mutant oligonucleotide (167A) to exon 1 PCR products from patients 458, 1396 and 31 controls. Fig. 10D is the hybridization of normal oligonucleotide (167G) to stripped dot blot from C. None of the 62 control chromosomes carried this mutation.

The second mutation in patient 1396 was identified in exon 6: a heterozygous C→T substitution (bp 1081) that converted an arginine residue to a cysteine residue, and abolished a *Hha*I restriction endonuclease site. Restriction analysis confirmed the heterozygous substitution in 1396 (Fig. 9D) and showed that 50 control chromosomes were negative. Fig. Tooth decay does not occur in patients having a saliva above pH 5.0. 9D is the HhaI restriction analysis of exon 6 PCR products for patient 1396 and 2 controls (C). Expected fragments: control allele, 152, 86, 13 bp; mutant allele 165, 86 bp. The 13-bp fragment has been run off the gel.

(4) Additional sequence changes

*Hha*I analysis of exon 6, mentioned above, revealed a DNA polymorphism. This change is a T→C substitution at bp 1068 which does not alter the amino acid (serine), but creates a *Hha*I recognition site. T at bp 1068 was found in 9% of tested chromosomes. Sequence analysis identified 2 discrepancies with the published cDNA sequence: a G→A substitution at bp 542 which converts the glycine to an aspartate codon, and a C→T change at bp 1032 which does not alter the amino acid (threonine). Since all DNAs tested (>50 chromosomes) carried the A at bp 542 and the T at bp 1032, it is likely that the sequence of the original cDNA contained some cloning artifacts.

### Genotype:phenotype correlation

Table 2 summarizes the current status of mutations in severe MTHFR deficiency. In 8 patients, both mutations have been identified; in 5 patients (3 families), only 1 mutation has been identified. Overall the correlation between the genotype, enzyme activity, and phenotype is quite consistent. Five patients, with onset of symptoms within the first year of life, had ≤ 3% of control activity. Three of these patients had missense mutations in the homozygous state: two patients with the threonine to methionine substitution (C692T) and one patient with the proline to leucine substitution (C764T). The nonsense mutation (C559T) in the homozygous state in patients 1554 and 1627 (previously reported in Goyette P et al., Nature Genetics, 1994, 7:195-200) is also associated with a neonatal severe form, as expected.

The other patients in Table 2 had ≥ 6% of control activity and onset of symptoms within or after the 2nd decade of life; the only exception is patient 1834, as previously reported (Goyette P et al., Nature Genetics, 1994, 7:195-200). The three patients (356, 458 and 1396) with missense mutations (G167A, C985T, C1015T and C1081T) are similar to those previously reported (patients 1779, 1834 and 1863) who had an arginine to glutamine substitution and a second unidentified mutation (Goyette P et al., Nature *Genetics,* 1994, 7:195-200). The sisters with the 5' splice mutation and an unidentified second mutation also had levels of activity in the same range and onset of symptoms in the second decade, but the activity is likely due to the second unidentified allele.

### Discussion

The patients come from diverse ethnic backgrounds. Although patients 1554 and 1627 are both Native Americans, the mutations occur on different haplotypes, suggesting recurrent mutation rather than identity by descent. Since the substitution occurs in a CpG dinucleotide, a "hot spot" for mutation, recurrent mutation is a reasonable hypothesis. It is difficult to assess whether some mutations are population-specific since the numbers are too small at the present time.

MTHFR deficiency is the most common inborn error of folate metabolism, and a major cause of hereditary homocysteinemia. The recent isolation of a cDNA for MTHFR has · permitted mutational analysis at this locus, with the aims of defining important domains for the enzyme and of correlating genotype with phenotype in MTHFR-deficient patients.

The 7 mutations described here (6 single amino acid substitutions and a 5' splice site mutation) bring the total to 9 mutations identified thus far in severe MTHFR deficiency and complete the mutation analysis for 8 patients. The identification of each mutation in only one or two families points to the striking degree of genetic heterogeneity at this locus. Seven of the 9 mutations are located in CpG dinucleotides, which are prone to mutational events.

### 5' splice site mutation

The G→A substitution at the GT dinucleotide of the 5' splice site in patients 354 and 355 results in a 57bp in-frame deletion of the coding sequence, which should delete 19 amino acids of the protein. This deletion occurs as a result of the activation of a cryptic 5' splice site (AG/gc) even though this cryptic site does not have a perfect 5' splice site consensus sequence (AG/gt). However, GC (instead of GT) as the first 2 nucleotides of an intron has been reported in several naturally-occurring splice sites, such as in the genes for human prothrombin and human adenine phosphoribosyltransferase and twice within the gene for the largest subunit of mouse RNA polymerase II. The remaining nucleotides of the cryptic site conform to a normal splice site consensus sequence with its expected variations ($A_{60}$ $G_{79}$/ $g_{100}t_{100}a_{59}a_{71}g_{82}t_{50}$). It is unlikely that the deleted enzyme resulting from this aberrantly-spliced mRNA would have any activity; 8 of the 19 deleted amino acids are conserved in the *E. Coli* enzyme. Although the 2 patients show residual enzyme activity in the range of 20% of controls, the activity is probably due to the unidentified second allele in these patients.

### 6 missense mutations

The Arg→Cys substitution (C1081T) in patient 1396 is within a hydrophilic sequence previously postulated to be the linker region between the catalytic and regulatory domains of MTHFR (Goyette P et al., Nature Genetics, 1994, 7:195-200). These 2 domains are readily separable by mild trypsinization of the porcine enzyme. The linker domain, a highly-charged region, is likely to be located on the outside surface of the protein and therefore more accessible to proteolysis. Because the Arg→Cys substitution converts a charged hydrophilic residue to an uncharged polar residue, it cannot be considered a conservative change, and could affect the stability of the enzyme.

The 2 Arg→Cys substitutions identified in patients 356 and 458 (C985T and C1015T, respectively) may be involved in binding the FAD cofactor. Previous work in the literature showed that heating fibroblast extracts at 55°, in the absence of the FAD cofactor, inactivated MTHFR completely. The addition of FAD to the reaction mixture before heat inactivation restored some enzyme activity to control extracts and to extracts from some patients, while the extracts of patients 356 and 458 were unaffected. Based on these observations, it was suggested that these 2 patients had mutations affecting a region of the protein involved in binding FAD. The 2 mutations are found in close proximity to each other, within 11 amino acids. In patient 356, the Arg residue is evolutionarily-conserved in *E. Coli* and is found in a stretch of 9 conserved amino acids, suggesting a critical role for this residue; the altered Arg residue in patient 458 is not evolutionarily-conserved. Crystal structure analysis of medium chain acyl-CoA dehydrogenase (MCAD), a flavoprotein, has defined critical residues involved in the binding of FAD. Two consecutive residues of the MCAD protein, Met165 and Trp166, involved in interactions with FAD, can also be identified in MTHFR, 3 and 4 amino acids downstream, respectively, from the Arg residue altered in patient 458.

The Thr→Met substitution (C692T) is found in a region of high conservation with the *E. Coli* enzyme and in a region of good homology with human dihydrofolate reductase (DHFR) (Fig. 11). In Fig. 11, = is identity; • is homology; and $ is identity to bovine DHFR enzyme. An asterisk (*) indicates location of Thr→Met substitution. Considering the early-onset phenotype of the patients, one can assume that the threonine residue is critical for activity or that it contributes to an important domain of the protein. This region of homology in DHFR contains a residue, Thr136, which has been reported to be involved in folate binding. This Thr residue in DHFR aligns with a Ser residue in MTHFR, an amino acid with similar biochemical properties. The Thr → Met substitution is located 8 amino acids downstream from this Ser codon, in the center of the region of homology between the 2 enzymes. It is therefore hypothesized that the Thr → Met substitution may alter the binding of the folate substrate.

The G167A (Arg → Gln) and C764T (Pro → Leu) substitutions both affect non-conserved amino acids. Their importance in the development of MTHFR deficiency cannot be determined at the present time. All the mutations identified thus far are located in the 5' end of the coding sequence, the region thought to encode the catalytic domain of MTHFR. Mutation analysis has been useful in beginning to address the structure: function properties of the enzyme as well as to understand the diverse phenotypes in severe MTHFR deficiency.

### III. Identification of A→V mutation

SSCP analysis and direct sequencing of PCR fragments were used to identify a C to T substitution at bp 677, which converts an alanine residue to a valine residue (Fig. 5A). The primers for analysis of the A→V change are: 5'-TGAAGGAGAA GGTGTCTGCG GGA-3' (SEQ ID NO:13) (exonic) and 5'-AGGACGGTGC GGTGAGAGTG-3' (SEQ ID NO:14) (intronic); these primers generate a fragment of 198 bp. Fig. 5A depicts the sequence of two individuals, a homozygote for the alanine residue and a homozygote for the valine residue. The antisense strands are depicted. This alteration creates a *Hinf*I site (Fig. 5B), which was used to screen 114 unselected French Canadian chromosomes; the allele frequency of the substitution was .38. The substitution creates a *Hinf*I recognition sequence which digests the 198 bp fragment into a 175 bp and a 23 bp fragment; the latter fragment has been run off the gel. Fig. 5B depicts the three possible genotypes. The frequency of the 3 genotypes were as follows: -/-, 37%; +/-, 51%; and +/+, 12% (the (+) indicates the presence of the *Hinf*I restriction site and a valine residue).

The alanine residue is conserved in porcine MTHFR, as well as in the corresponding metF and *stymetF* genes of *E. Coli* and S. *Typhimurium*, respectively. The strong degree of conservation of this residue, and its location in a region of high homology with the bacterial enzymes, alluded to its importance in enzyme structure or function. Furthermore, the frequency of the (+/+) genotype was consistent with the frequency of the thermolabile MTHFR variant implicated in vascular disease.

### Clinical material

To determine the frequency of the A→V mutation, DNA from 57 individuals from Quebec was analyzed by PCR and restriction digestion. The individuals, who were all French Canadian, were not examined clinically or biochemically.

The 40 individuals analyzed in Table 3 had been previously described in Engbersen et al. (Am. J. Hum. *Genet.*, 1995, 56:142-150). Of the 13 cardiovascular patients, 8 had cerebrovascular arteriosclerosis and 5 had peripheral arteriosclerosis. Five had thermolabile MTHFR while 8 had thermostable MTHFR (greater than 33% residual activity after heating). Controls and patients were all Dutch-Caucasian, between 20-60 years of age. None of these individuals used vitamins which could alter homocysteine levels. Enzyme assays and homocysteine determinations were also reported by Engbersen et al. (*Am. J. Hum. Genet.,* 1995, 56:142-150).

Table 3

| Correlation between MTHFR genotype and enzyme activity, thermolability and plasma homocysteine level | | | |
|---|---|---|---|
| | -/- n=19 | +/- n=9 | +/+ n=12 |
| **specific activity[a,b] (nmol $CH_20$/mg.protein/hr)** | 22.9± 1.7 (11.8 - 33.8) | 15.0 ± 0.8 (10.2-18.8) | 6.9 ± 0.6 (2.6-10.2) |
| **residual activity after heating[a,b] (%)** | 66.8 ± 1.5 (55-76) | 56.2 ± 2.8 (41-67) | 21.8 ± 2.8 (10-35) |
| **plasma homocysteine [a,c] (μM)(after fasting)** | 12.6 ± 1.1 (7-21) | 13.8 ± 1.0 (9.6-20) | 22.4 ± 2.9 (9.6-42) |
| **plasma homocysteine [a,c] (μM)(post-methionine load)** | 41.3 ± 5.0[d] (20.9 -110) | 41 ± 2.8 (29.1-54) | 72.6 ± 11.7[e] (24.4-159) |

[a] one-way anova p<.01
[b] paired t test for all combinations p<.01
[c] paired t test p<.05 for +/+ group versus +/- group or -/- group; p>.05 for +/- versus -/- group.
[d] n=18 for this parameter
[e] n=11 for this parameter

Enzyme activity and plasma homocysteine were determined as previously reported. Each value represents mean ± standard error. The range is given in parentheses below the mean.

**Correlation of A→V mutation with altered MTHFR function**

A genotypic analysis was performed and enzyme activity and thermolability were measured in a total of 40 lymphocyte pellets from patients with premature vascular disease and controls. 13 vascular patients were selected from a previous study (Engbersen et al., *Am. J. Hum. Genet.,* 1995, 56:142-150), among which 5 were considered to have thermolabile MTHFR. From a large reference group of 89 controls, all 7 individuals who had thermolabile MTHFR were studied, and an additional 20 controls with normal MTHFR were selected from the same reference group. Table 3 documents the relationship between genotypes and specific enzyme activity, thermolability and plasma homocysteine level. The mean MTHFR activity for individuals homozygous for the substitution (+/+) was approximately 30% of the mean activity for (-/-) individuals, homozygous for the alanine residue. Heterozygotes had a mean MTHFR activity that was 65% of the activity of (-/-) individuals; this value is intermediate between the values for (-/-) and (+/+) individuals. The ranges of activities showed some overlap for the heterozygous and (-/-) genotypes, but homozygous (+/+) individuals showed virtually no overlap with the former groups. A one-way analysis of variance yielded a p value <.0001; a pairwise Bonferroni t test showed that all three genotypes were significantly different with p<0.01 for the three possible combinations.

The three genotypes were all significantly different (p<.01) with respect to enzyme thermolability. The mean residual activity after heat inactivation for 5 minutes at 46° was 67%, 56% and 22% for the (-/-), (+/-) and (+/+) genotypes, respectively. While the degree of thermolability overlaps somewhat for (-/-) individuals and heterozygotes, individuals with two mutant alleles had a distinctly lower range. Every individual with the (+/+) genotype had residual activity <35% after heating, and specific activity <50% of that of the (-/-) genotype.

Total homocysteine concentrations, after fasting and 6 hours after methionine loading, were measured in plasma by high performance liquid chromatography using fluorescence detection. Fasting homocysteine levels in (+/+) individuals were almost twice the value for (+/-) and (-/-) individuals. The differences among genotypes for plasma homocysteine were maintained when homocysteine was measured following 6 hours of methionine loading. A one-way anova yielded a p < .01 for the fasting and post-methionine homocysteine levels. A pairwise Bonferroni t test showed that only homozygous mutant individuals had significantly elevated homocysteine levels (p<.05).

**PCR-based mutagenesis for expression of A→V mutation in vitro**

PCR-based mutagenesis, using the cDNA-containing Bluescript™ vector as template, was used to create the A to V mutation. Vent™ polymerase (NEB) was used to reduce PCR errors. The following primers were used: primer 1, bp -200 to -178, sense; primer 2, bp 667 to 687, antisense, containing a mismatch, A, at bp 677; primer 3, 667 to 687, sense, containing a mismatch, T, at bp 677; primer 4, bp 1092 to 1114, antisense. PCR was performed using primers 1 and 2 to generate a product of 887 bp, and using primers 3 and 4 to generate a product of 447 bp. The two PCR fragments were isolated from a 1.2% agarose gel by Geneclean™ (BIO 101). A final PCR reaction, using primers 1 and 4 and the first 2 PCR fragments as template, was performed to generate a 1.3 kb band containing the mutation. The 1.3 kb fragment was digested with NcoI and MscI, and inserted into the wild-type cDNA- containing expression vector by replacing the sequences between the NcoI site at bp 11 and the MscI site at bp 943. The entire replacement fragment and the cloning sites were sequenced to verify that no additional changes were introduced by PCR.

**Expression analysis of wild-type and mutagenized cDNA**

Overnight cultures of JM105™ containing vector alone, vector + wild-type MTHFR cDNA, or vector + mutagenized cDNA were grown at 37° C. in 2 x YT media with .05 mg/ml ampicillin. Fresh 10 ml. cultures of each were inoculated with approximately 50 µL of overnight cultures for a starting O.D. of 0.05, and were grown at 37° C to an O.D. of 1 at 420 nM. Cultures were then induced for 2 hrs. with 1 mM IPTG and pelleted. The cells were resuspended in TE buffer with 2 µg/ml aprotinin and 2µg/ml leupeptin (3.5 x wet weight of cells). Cell suspensions were sonicated on ice for 3 x 15 sec. to break open cell membranes and then centrifuged for 30 min. at 4°C. to pellet cell debris and unlysed cells. The supernatant was removed and assayed for protein concentration with the Bio-Rad™ protein assay. Western analysis was performed using the Amersham ECL™ kit according to the instructions of the supplier, using antiserum generated against purified porcine liver MTHFR. Enzymatic assays were performed by established procedures; pre-treating the extracts at 46°C. for 5 min. before determining activity assessed thermolability. Specific activities (nmol formaldehyde/hr./mg. protein) were calculated for the 2 cDNA-containing constructs after subtraction of the values obtained with vector alone (to subtract background *E. Coli* MTHFR activity).

The MTHFR cDNA (2.2 kb) (Fig. 6) has an open reading frame of 1980 bp, predicting a protein of 74.6 kDa. The purified porcine liver enzyme has been shown to have subunits of 77 kDa. Western analysis (Fig. 7A) of several human tissues and of porcine liver has revealed a polypeptide of 77 kDa in all the studied tissues, as well as an additional polypeptide of approximately 70 kDa in human fetal liver and in porcine liver, suggesting the presence of isozymes. Two µg of bacterial extract protein was used for lanes 1-3. The tissues (lanes 4-8) were prepared by homogenization in .25M sucrose with protease inhibitors (2 µg/ml each of aprotinin and leupeptin), followed by sonication (3 x 15 sec.) on ice. The extracts were spun for 15 min. in a microcentrifuge at 14,000 g and 100 µg of supernatant protein was used for Western analysis. h=human;.p=porcine.

The wild-type cDNA and a mutagenized cDNA, containing the A→V substitution, were expressed in *E. Coli* to yield a protein of approximately 70 kDa (Fig. 7A), which co-migrates with the smaller polypeptide mentioned above. Treatment of extracts at. 46°C for 5 minutes revealed that the enzyme containing the substitution was significantly more thermolabile than the wild-type enzyme ($p<.001$; Fig. 7B). Two separate experiments (with 3-4 replicates for each construct for each experiment) were performed to measure thermostable activity of the wild-type MTHFR and mutagenized MTHFR A→V cDNAs. The values shown represent mean ± standard error for each experiment, as % of residual activity after heating. The means of the specific activities before heating (expressed as nmol formaldehyde/hr./mg. protein) were as follows: Exp. 1, 3.8 and 5.3 for MTHFR and MTHFR A→V, respectively; Exp. 2, 6.2 and 7.5 for MTHFR and MTHFR A→V, respectively. The expression experiments were not designed to measure differences in specific activity before heating, since variation in efficiencies of expression could contribute to difficulties in interpretation. Curiously though, the specific activity for the mutant construct was higher in both experiments. It is possible that the mutant protein has increased stability in *E. Coli,* or that inclusion bodies in the extracts contributed to differences in recovery of properly-assembled enzyme.

These studies have identified a common substitution in the MTHFR gene which results in thermolability in vitro and in vivo. The mutation, in the heterozygous or homozygous state, correlates with reduced enzyme activity and increased thermolability of MTHFR in lymphocyte extracts. A significant elevation in plasma homocysteine was observed in individuals who were homozygous for the mutation. Statistically-significant differences for homocysteine levels were not observed between heterozygotes and (-/-) individuals; this observation is not surprising, since plasma homocysteine can be influenced by several environmental factors, including intake of folate, vitamin $B_{12}$, vitamin $B_6$, and methionine, as well as by genetic variation at other loci, such as the cystathionine-β-synthase gene.

The alanine to valine substitution conserves the hydrophobicity of the residue and is associated with small changes in activity, in contrast to non-conservative changes, such as the previously-reported arginine to glutamine change in MTHFR, which is associated with a greater decrease in enzyme activity and severe hyperhomocysteinemia. The alanine residue is situated in a region of homology with the bacterial metF genes. The same region of homology was also observed in the human dihydrofolate reductase (DHFR) gene (Fig. 11), although the alanine residue itself is not conserved; this region of amino acids 130-149 of DHFR contains T136 which has been implicated in folate binding in an analysis of the crystal structure of recombinant human DHFR. It is tempting to speculate that this region in MTHFR is also involved in folate binding and that the enzyme may be stabilized in the presence of folate. This hypothesis is compatible with the well-documented influence of folate on homocysteine levels and with the reported correction of mild hyperhomocysteinemia by folic acid in individuals with premature vascular disease, and in individuals with thermolabile MTHFR.

Although the cDNA is not long enough to encode the larger MTHFR polypeptide, it is capable of directing synthesis of the smaller isozyme. The ATG start codon for this polypeptide is within a good consensus sequence for translation initiation. Whether the isozyme is restricted to liver and what its role is in this tissue remain to be determined.

These data have identified a common genetic change in MTHFR which results in thermolability; these experiments do not directly address the relationship between this change and vascular disease. Nonetheless, this polymorphism represents a diagnostic test for evaluation of MTHFR thermolability in hyperhomocysteinemia. Large case-control studies are required to evaluate the frequency of this genetic change in various forms of occlusive arterial disease and to examine the interaction between this genetic marker and dietary factors. Well-defined populations need to be examined, since the limited data set thus far suggests that population-specific allele frequencies may exist. More importantly, however, the identification of a candidate genetic risk factor for vascular disease, which may be influenced by nutrient intake, represents a critical step in the design of appropriate therapies for the homocysteinemic form of arteriosclerosis.

## cDNA FOR MTHFR AND ITS POTENTIAL UTILITY

The cDNA sequence is a necessary starting point for the detection of MTHFR sequence abnormalities that would identify individuals at risk for cardiovascular and neurological diseases, as well as other disorders affected by folic acid metabolism. Diagnostic tests by DNA analysis are more efficient and accurate than testing by enzymatic/biochemical assays. Less blood is required and results are available in a shorter period of time. The tests could be performed as a routine operation in any laboratory that performs molecular genetic diagnosis, without the specialized reagents/expertise that is required for an enzyme-based test.

The second major utility of the cDNA would be in the design of therapeutic protocols, for correction of MTHFR deficiency. These protocols could directly involve the gene, as in gene therapy trials or in the use of reagents that could modify gene expression. Alternatively, the therapy might require knowledge of the amino acid sequence (derived from the cDNA sequence), as in the use of reagents that would modify enzyme activity. The identification of sequences and/or sequence changes in specific regions of the cDNA or protein, such as FAD binding sites or folate-binding sites, are useful in designing therapeutic protocols involving the above nutrients.

## UTILITY OF INVENTION IN CLINICAL AND DIAGNOSTIC STUDIES

Coronary artery disease patients in Montreal (n=153) were studied to examine the frequency of the alanine to valine substitution. Fourteen percent of the patients were homozygous for this mutation. An analysis of 70 control individuals (free of cardiovascular disease) demonstrated that only seven % of these individuals were homozygous for the alanine to valine mutation.

Analysis of homocysteine levels in 123 men of the above patient group indicated that the mutant allele significantly raised homocysteine levels from 10.2 micromoles/L in homozygous normal men to 11.5 and 12.7 in heterozygotes and homozygous mutants, respectively.

Families with a child with spina bifida, a neural tube defect, have been examined for the presence of the alanine to valine mutation. Approximately 16% of mothers who had a child with spina bifida were homozygous for this mutation, while only 5%. of control individuals were homozygous. Fathers of children with spina bifida also had an increased prevalence of the homozygous mutant genotype (10%) as did the affected children themselves (13%).

Table 4 indicates the interactive effect of folic acid with the homozygous mutant alanine to valine change. In a study of families from Framingham, Massachusetts and Utah, individuals who were homozygous mutant but had folate levels above 5 ng/ml did not have increased homocysteine levels compared to individuals with the normal or heterozygous genotype. However, individuals who were homozygous mutant but had folate levels below 5 ng/ml had homocysteine levels that were significantly higher than the other genotypes.

Table 4

| Mean fasting and PML homocysteine levels for different MTHFR genotypes | | | | |
|---|---|---|---|---|
| Plasma Homocysteine | MTHFR genotype | | | |
| | Normals (-/-) | Heterozygote s (+/-) | Homozygotes (+/+) | $P_{trend}$ |
| N | 58 | 61 | 30 | |
| Fasting* | 9.4 * | 9.2 | 12.1 | 0.02 |
| Folate <5 ng/mL | 10.2 | 10.4 | 15.2 | 0.002 |
| Folate $^3$ 5ng/mL | 8.2 | 7.5 | 7.5 | 0.52 |
| Post-Methionine load | 30.0 | 30.9 | 31.3 | 0.62 |

* Significant interaction between folate levels and genotype (p=0.03)

Table 4 provides preliminary data for therapeutic intervention by folic acid supplementation to individuals who are homozygous for the alanine to valine change. The data suggest that higher levels of plasma folate would lead to normalization of homocysteine levels in mutant individuals and might prevent the occurrence of disorders associated with high homocysteine levels, such as cardiovascular disease, neural tube defects, and possibly other disorders. Folic acid supplementation for mutant individuals might also restore methionine and S-adenosylmethionine levels to normal. This would be relevant for disorders that are influenced by methylation, such as neoplasias, developmental anomalies, neurologic disease, etc.

**Genetic polymorphism in methylenetetrahydrofolate reductase (MTHFR) associated with decreased activity**

A common mutation (C677T) results in a thermolabile enzyme with reduced specific activity (approximately 35% of control values in homozygous mutant individuals). Homozygous mutant individuals (approximately 10% of North Americans) are predisposed to mild hyperhomocysteinemia, when their folate status is low. This genetic-nutrient interactive effect is believed to increase the risk for neural tube defects (NTD) and vascular disease. There has been reported an increased risk for spina bifida in children with the homozygous mutant genotype for C677T. With the present invention, a second common variant in MTHFR (A1298C), an E to A substitution has been characterized. Homozygosity was observed in approximately 10% of Canadian individuals. This polymorphism was associated with decreased enzyme activity; homozygotes had approximately 60% of control activity in lymphocytes.

A sequence change (C1298A) has been identified. Heterozygotes for both the C677T and the A1298C mutation, approximately 15% of individuals, had 50%-60% of control activity, a value that was lower than that seen in single heterozygotes for the C677T variant. No individuals were homozygous for both mutations. A silent genetic variant T1317C, was identified in the same exon. It was relatively infrequent (allele frequency = 5%) in the study group, but was common in a small sample of African individuals (allele frequency = 39%).

In addition, by virtue of the role of MTHFR in folate-dependent homocysteine metabolism, the C677T mutation predisposes to mild hyperhomocysteinemia, a risk factor for vascular disease, in the presence of low folate status. By the present invention, the frequency of the A1298C variant has been determined and its potential impact on enzyme function has been assessed.

Patients with spina bifida and mothers of patients were recruited from the Spina Bifida Clinic at the Montreal Children's Hospital following approval from the Institutional Review Board. Control children and mothers of controls were recruited from the same institution. Blood samples were used to prepare DNA from peripheral leukocytes, to assay MTHFR activity in lymphocyte extracts, and to measure total plasma homocysteine (tHcy). The presence of the C677T mutation (A to V) was evaluated by PCR and HinfI digestion (2). The A1298C mutation was initially examined by PCR and MboII digestion (5). The silent mutation, T1317C, was identified by SSCP and sequence analysis in a patient with severe MTHFR deficiency and homocystinuria. This patient, an African-American female, already carries a previously-described splice mutation (patient 354 (8)). Since this mutation also creates a MboII site and results in a digestion pattern identical to that of the A1298C mutation, distinct artificially-created restriction sites were used to distinguish between these 2 mutations. Detection of the A1298C polymorphism was performed with the use of the sense primer 5'-GGGAGGAGCTGACCAGTGCAG-3' and the antisense primer (5'-GGGGTCAGGCCAGGGGCAG-3'), such that the 138bp PCR fragment was digested into 119bp and 19bp fragments by Fnu4HI in the presence of the C allele. An antisense primer (5'-GGTTCTCCCGAGAGGTAAAGATC-3'), which introduces a TaqI site, was similarly designed to identify the C allele of the T1317C polymorphism. Together with a sense primer (5'-CTGGGGATGTGGTGGCACTGC-3'), the 227bp fragment is digested into 202bp and 25bp fragments.

Table 5

| Genotype distributions, MTHFR activity (nmol formaldehyde/mg protein/hour), and total plasma homocysteine (tHcy;μM) for mothers and children | | | | | | | | | |
|---|---|---|---|---|---|---|---|---|---|
| Mothers (n=141) | | | | | | | | | |
| | E/E | | | E/A | | | A/A | | |
| | A/A | A/V | V/V | A/A | A/V | V/V | A/A | A/V | V/V |
| # | 24 | 32 | 19 | 27 | 26 | 0 | 13 | 0 | 0 |
| % | 17 | 23 | 13 | 19 | 18 | 0 | 9 | 0 | 0 |
| MTHFR | 49.0±18.9 [14] | 33.0±10.8 [19]* | 15.7±4.5 [11]* | 45.0±16.0 [15] | 30.2±19.3 [15]* | - | 32.1±9.0 [7]* | - | - |
| THcy | 9.5±3.1 [24] | 10.0±3.2 [32] | 12.2±7.1 [19]** | 8.4±2.1 [25] | 10.0±3.1 [26] | - | 9.5±2.0 [13] | - | - |
| Children (n=133) | | | | | | | | | |
| # | 23 | 43 | 18 | 20 | 15 | 1 | 13 | 0 | 0 |
| % | 17 | 32 | 13 | 15 | 11 | 1 | 10 | 0 | 0 |
| MTHFR | 52.0±17.0 [12] | 38.2±15.0 [27]* | 16.2±5.3 [11]* | 35.7±9.7 [18]* | 26.1±5.0 [9]* | 21.6 [1] | 29.5±10.3 [6]* | - | - |
| THcy | 7.6±2.5 [23] | 8.2 ±3.0 [43] | 9.7±5.1 [18]** | 7.5±2.3 [20] | 8.1±2.8 [15] | 9.5 [1] | 7.4±1.5 [13] | - | - |
| The three A1298C genotypes and the three C677T genotypes are designated by the amino acid codes: EE, EA, AA, and AA, AV, VV, respectively. Statistical significance was assessed by student t-test, in comparison with EEAA values. | | | | | | | | | |

* ($p<0.05$);

** ($p\leq0.07$). Standard deviations are given and square brackets indicate the number of individuals for whom MTHFR activities and homocysteine levels were available.

The frequencies of the three genotypes for the A1298C mutation (EE, EA and AA) were not different between case and control mothers, or between case and control children (data not shown). Consequently, all the mothers and all the children were grouped together for analyses (Table 5). Nine % of mothers had the homozygous AA genotype while 37% were heterozygous. This frequency is quite similar to the frequency of the homozygous mutant genotype (VV) for the C677T polymorphism. In the MTHFR human cDNA sequence mentioned above, the cDNA contained the C nucleotide at bp 1298 change as a C1298A substitution. Since the A nucleotide is clearly the more frequent base at this position, the A1298C nomenclature was chosen.

Since the C677T mutation (A to V) decreases MTHFR activity and increases homocysteine levels, the three genotype groups for the A1298C (E to A) mutation were further stratified by the genotype for the A to V mutation, to avoid the confounding influence of the latter polymorphism on MTHFR activity and homocysteine levels. The frequencies of the 9 genotypes, with MTHFR activity and homocysteine levels for each genotype, are shown in Table 5. If the mothers and children without either mutation i.e. EE/AA are used as the reference (control) group, the mothers and children that are homozygous for the A1298C mutation (AAAA) have approximately 65% and 57%, respectively, of control MTHFR activity. Heterozygotes for the C677T change alone (EEAV) have approximately 70% of control activity, as reported in other studies, while double heterozygotes (EAAV), 18% of mothers and 11% of children, have an additional loss of activity (approximately 62% and 50% of control values, respectively).

Homocysteine levels were not significantly increased by the A1298C mutation, but homocysteine was elevated (with borderline significance, $p\leq0.07$) in mothers and children who were homozygous for the C677T change. The small number of individuals who were homozygous for the A1298C mutation (n=13) may have influenced the power of the statistical analyses and precluded an investigation of the genetic-nutrient interactive effect that leads to mild hyperhomocysteinemia, as seen in individuals with the C677T mutation.

The T1317C substitution does not alter the amino acid (phenylalanine) and is likely a benign change, although a splicing defect cannot be ruled out at the present time. In an evaluation of 38 control mothers from this study, 2 were found to be heterozygous and one was identified as a homozygote, resulting in an allele frequency of 5% (4/76). Since this substitution was identified in an African-American female, control African individuals were also examined (n=9). Seven of these were heterozygous, resulting in an allele frequency of 39% (7/18).

The A1298C mutation clearly reduces MTHFR activity, albeit to a lesser extent than the C677T mutation.

Consequently its effect on homocysteine levels is also attenuated and, in fact, may only be significant when an individual carries both mutations and/or has poor nutrient status. However, since double heterozygotes are estimated to represent approximately 15% of the population, this variant should be examined in conjunction with the C677T variant in studies of hyperhomocysteinemia.

The A1298C mutation is clearly polymorphic in Canadian individuals and should be examined in other populations. The A nucleotide is likely to be the ancestral sequence since it represents the more common allele, although the original human MTHFR cDNA sequence (GenBank accession number U09806) carried the C nucleotide. This polymorphism is similar in frequency to the C677T polymorphism. Presumably the two substitutions arose separately on a A1298/C677 or E/A haplotype, since the haplotype with both substitutions (C1298/T677 or A/V) is extremely rare. One such haplotype was seen in a child with the EAVV genotype, suggesting a recombinant chromosome.

Doubly homozygous individuals (AAW) were not observed in this study. Since the double mutation in cis is rare, it is possible that not enough alleles were studied. Larger studies in other populations might result in the identification of these individuals. Presumably the MTHFR activity would be even lower and homocysteine levels might be higher than those observed thus far.

The C677T polymorphism in exon 4 is within the N-terminal catalytic domain of the enzyme whereas the A1298C polymorphism in exon 7 is within the C-terminal regulatory domain. The more dramatic effect on enzyme activity with the first polymorphism may be a consequence of its location within the catalytic region. The second polymorphism could affect enzyme regulation, possibly by S-adenosylmethionine, an allosteric inhibitor of MTHFR, which is known to bind in the C-terminal region.

Many studies have examined the effects of the C677T polymorphism on MTHFR enzyme activity and on homocysteine levels. Although the correlation between the presence of this substitution and decreased enzyme activity/increased homocysteine levels has been quite good, the variability in results, particularly in heterozygous individuals, may reflect the presence of a second common variant in the population.

The third variant, T1317C, was present on 5% of alleles in Canadian individuals but appears to be extremely common in individuals of African ancestry. The methodology outlined in this report should be used to assess the frequency of the A1298C and T1317C in other populations, since the use of the MboII restriction site for analysis of the A1298C change, as first reported, would not discriminate between the 2 polymorphisms.

The C677T mutation is a risk factor for hyperhomocysteinemia and has been implicated in both neural tube defects and vascular disease.

**Gene structure of human and mouse methylenetetrahydrofolate reductase (MTHFR)**

A human cDNA for MTHFR, 2.2 kb in length, has been expressed and shown to result in a catalytically-active enzyme of approximately 70 kDa. Fifteen mutations have been identified in the MTHFR gene: 14 rare mutations associated with severe enzymatic deficiency and one common variant associated with a milder deficiency. The common polymorphism has been implicated in three multifactorial diseases: occlusive vascular disease, neural tube defects and colon cancer. The human gene has been mapped to chromosomal region 1p36.3 while the mouse gene has been localized to distal Chromosome 4. The isolation and characterization of the human and mouse genes for MTHFR is herein reported. A human genomic clone (17 kb) was found to contain the entire cDNA sequence of 2.2 kb; there were 11 exons ranging in size from 102 bp to 432 bp. Intron sizes ranged from 250 bp to 1.5 kb with one exception of 4.2 kb. The mouse genomic clones (19 kb) start 7 kb 5' to exon 1 and extend to the end of the coding sequence. The mouse amino acid sequence is approximately 90% identical to the corresponding human sequence. The exon sizes, locations of intronic boundaries, and intron sizes are also quite similar between the two species. The availability of human genomic clones has been useful in designing primers for exon amplification and mutation detection. The mouse genomic clones may be used to make constructs for gene targeting and generation of mouse models for MTHFR deficiency.

A common polymorphism, C677T has been identified, which converts an alanine codon to valine (Frosst et al., 1995). This common polymorphism, which is present on approximately 35% of alleles in the North American population, encodes the thermolabile variant and predisposes to mild hyperhomocysteinemia when folate status is low (Frosst et al., 1995; Jacques et al., 1996; Christensen et al., 1997). This genetic-nutrient interactive effect is believed to be a risk factor for arteriosclerosis (Frosst et al, 1995) and neural tube defects. In contrast, the mutant homozygous genotype may decrease the risk for colon cancer.

The characterization of the genomic structure for human MTHFR is reported herein. The corresponding analysis of the mouse gene, with a comparison of the overall organization of the gene and the amino acid sequences in these two species, is also shown.

**Screening of genomic libraries**

Genomic libraries were screened using standard methods of plaque hybridization. The 2.2 kb human cDNA was radiolabelled and used as a probe in screening both human and murine genomic libraries. Screening for the human gene was performed on a phage library of partial EcoRI digestion fragments from total genomic DNA (ATCC # 37385), and on a phage library of chromosome 1-specific complete EcoRI digestion fragments (ATCC# 57738). Screening for the mouse gene was performed on a λDASH library of partial Sau3A digestion fragments from total genomic DNA of mouse strain 129SV (obtained from Dr. J. Rossant, University of Toronto). Positive clones were purified by sequential rounds of screening and isolation, and phage DNA was isolated using phage DNA isolation columns (QIAGEN). Human clones were digested with EcoRI to release the inserts, and then with XbaI to facilitate cloning into Bluescript plasmid (Stratagene). The mouse clones were digested with SalI or EcoRI, and the inserts were subcloned into Bluescript.

**Characterization of mouse cDNA sequences**

Mouse genomic clones were sequenced (Sequenase kit, Amersham) using human cDNA primers spanning most of the available 2.2 kb cDNA. These sequences were then used to generate mouse-specific cDNA primers. The mouse-specific primers were used in PCR amplification of overlapping cDNA fragments from reverse-transcribed mouse liver RNA. The PCR products were subcloned into the PCRII vector (Invitrogen) and sequenced. Two different species of mouse (C57B1/6J and ct) were used to generate MTHFR sequence by RT-PCR, to ensure that the PCR protocol did not generate sequencing errors.

**Characterization of intron boundaries and sizes, and restriction analysis of human and mouse genes**

Primers from cDNA sequences of human and mouse were used to sequence the respective genomic clones. Intron boundaries were determined from regions of divergence between cDNA and genomic clone sequences, and by the identification of splice acceptor and donor consensus sites. Intronic sequences were obtained for 40-50 bp from the junctions and are shown in Figs. 12A-12B (human) and Figs. 13A-13B (mouse). The same cDNA primers were used in PCR amplification of total genomic DNA and of genomic clones to determine the approximate sizes of introns in the human and mouse genes. Table 6 lists the locations and approximate sizes of introns for both species. The PCR products were analyzed by restriction enzyme digestion to generate a preliminary restriction map of the gene. This restriction map was then confirmed by restriction analysis of the genomic clones in Bluescript.

Referring to Figs. 12A-12B, the bp location of the exons within the cDNA, in parentheses, is based on the published human cDNA sequence (GenBank accession number U09806). Bp 1 is 12 bp upstream from the ATG in the original cDNA; an asterisk indicates the equivalent base here. Exon 1 contains the ATG start site (underlined), and exon 11 contains the termination codon (underlined). Uppercase characters indicate exonic sequences, and lower case characters are intronic. Consensus splice junction sequences are underlined. The 3' boundary of exon 11 has been designated by the location of the polyA tail.

Referring to Figs. 13A-13B, the bp location of the exons within the cDNA, in parentheses, is based on the equivalent bp 1 of the human sequences in Figs. 12A-12B (bp 1 is indicated by an asterisk). Exon 1 contains the ATG start site (underlined), and exon 11 contains the termination codon (doubly underlined). Uppercase characters indicate exonic sequences, and lower case characters are intronic. Consensus splice junction sequences are underlined. The 3' boundary of exon 11 is designated as the termination codon, since the site of polyadenylation is unknown. Also underlined in exon 11 is the first repeat of the 52 bp repeated element.

Referring to Fig. 14, exon sizes for human and mouse are reported in Figs. 12A-12B and Figs. 13A-13B, respectively. Exons are indicated in shaded boxes. Uncharacterized regions of the gene are hatched, and exon numbering corresponds to Figs. 12A-12B and 13A-13B. E=EcoRI; X=XbaI; $A_n$=polyadenylation site. The EcoRI restriction site at the 5' end of the mouse gene is part of the phage polylinker sequence.

Referring to Fig. 15, residues that are identical to the human MTHFR sequence are shown as empty boxes, and gaps in amino acid homology are represented by a dash.

**Human genomic clones**

The genomic clones isolated from the human libraries contained a 16 kb EcoRI fragment, encompassing part of exon 1 and exons 2 through 11, and a 1 kb EcoRI fragment containing most of exon 1. Exon 1 is defined as the most 5' exon from the previously published cDNA sequence; it contains the ATG start site that was used to express the human cDNA in bacterial extracts (Frosst et al. 1995). A graphic representation of the human gene and its restriction map are depicted in Fig. 3. The sequences of each exon and 50 bp of flanking intronic sequences are shown in Figs. 12A-12B. Exons range in size from 102 bp to 432 bp, and the critical dinucleotides in the 5' and 3' splice site consensus sequences (GT and AG, respectively) are underlined. The 3' boundary of exon 11 is defined by the site of polyadenylation in the cDNA; a possible polyadenylation signal (AACCTA) is present 15 bp upstream of the polyadenylation site, although it varies from the consensus sequence. Table 6 lists the locations and approximate sizes of introns as determined by PCR amplification; the introns range in size from approximately 250 bp to 4.2 kb.

**Mouse genomic clones**

Genomic clones isolated from the mouse libraries were digested with EcoRI for subcloning and characterization. Exon nomenclature is based on the corresponding human gene sequences. Figs. 13A-13B list all known exons and their sizes, with 40 to 50 bp of flanking intronic sequences. Fig. 14 shows a graphic representation of the mouse genomic structure aligned with the human gene. The size of exon 11 is undetermined, since the sequence for this region was determined directly from the genomic clones. The termination codon is located within a region of 52 bp which is repeated 3 times in the gene. The significance of this, if any, is unknown at the present time. The dinucleotides of the splice junctions (underlined in Figs. 13A-13B) are in agreement with consensus sequences. Table 6 lists the approximate sizes of introns as determined by PCR, and their bp location in the cDNA. The introns range in size from approximately 250 bp to 4.2 kb.

**Comparison of the human and mouse genes**

The human and mouse genes are very similar in size and structure (Fig. 14). The introns are similar in size, and identical in location within the coding sequence. However, the mouse cDNA is one amino acid shorter in exon 1 which causes a shift in bp numbering of the mouse cDNA (Table 6, Figs. 13A-13B). Exon 1 was defined from the original published human cDNA, based on the presence of a translation start codon. In both human and mouse genes, the 5' boundary of exon 1 was assigned after the isolation of several non-coding cDNA extensions that are generated by alternative splicing from this junction. Characterization of these 5' cDNA extensions is in progress. The nucleotide sequences of the human and mouse genes are very similar within coding regions, but homology decreases dramatically in the 3' UTR region and within introns.

**Human and mouse primary amino acid sequence homology**

The primary amino acid sequences of human and mouse were compared to each other, and aligned with the sequence of the MetF (MTHFR) enzyme from bacteria (Fig. 15). The human and mouse amino acid sequences are almost 90% identical. As previously observed, only the 5' half of the mammalian sequences align with the bacterial enzyme; bacterial MTHFR has the same catalytic activity as the mammalian enzyme but lacks the regulatory region in the C-terminal domain. The murine amino acid sequence is two amino acids shorter than the human sequence: one less amino acid in exon 1 and one less in exon 11.

The isolation of the human MTHFR gene and the analysis of gene structure are part of an ongoing effort to study MTHFR deficiency in homocystinuria and in multifactorial diseases. The availability of genetic structure information and of intronic sequences will help in the mutational analysis of patients suffering from MTHFR deficiency and in the characterization of the 5' regulatory region.

Expression analysis of the 2.2kb cDNA in a bacterial expression system resulted in a catalytically-active 70kDa protein (Frosst et al. 1995). A MTHFR polypeptide of this size was observed in some human tissues on Western blots, but a larger isozyme (77 kDa), corresponding to the estimated size of the porcine polypeptide, was observed in all the examined tissues. These data suggested the presence of protein isoforms for MTHFR that could be tissue-specific (Frosst et al., 1995). Since human or mouse sequences homologous to the N-terminal porcine amino acid sequences have not been identified, it is assumed that the missing sequences required to encode the larger isoform are 5' to the available cDNA sequences. Two mRNAs for human MTHFR (approximately 7.5 and 8.5 kb) have been seen in all tissues on Northern blots (data not shown), suggesting very large UTRs. The isolation of 5' coding sequences has been complicated by the presence of several alternatively-spliced 5' non-coding extensions that splice into exon 1. The alternative splicing into exon 1 has been observed in both human and mouse MTHFR. The long UTRs and the alternative splicing events suggest that the regulation of this important gene may be quite complex.

Nonetheless, the available information has been critical for identification of mutations in patients with various forms of MTHFR deficiency. The mouse sequences in exons 1 and 2 have been useful in the design of antisense oligonucleotides to successfully inhibit MTHFR in mouse embryo cultures and disrupt development of the neural tube (Lanoue et al. 1997). The isolation and characterization of mouse genomic clones is essential for construction of targeting vectors to generate mouse models for MTHFR deficiency.

Table 6

| Approximate sizes of introns, and their locations in human and mouse MTHFR cDNA | | | |
|---|---|---|---|
| **Intron** | **Approximate size (kb)** | **Human location[1]** | **Mouse Location[1]** |
| 1 | 1.5 | 248-249 | 245-246 |
| 2 | 0.8 | 487-488 | 484-485 |
| 3 | 4.2 | 598-599 | 595-596 |
| 4 | 0.8 | 792-793 | 789-790 |
| 5 | 0.35 | 1043-1044 | 1040-1041 |
| 6 | 0.25 | 1178-1179 | 1175-1176 |
| 7 | 0.3 | 1359-1360 | 1356-1357 |
| 8 | 1.5 | 1542-1543 | 1539-1540 |
| 9 | 1.3 | 1644-1645 | 1641-1642 |
| 10 | 0.3 | 1764-1765 | 1761-1762 |

[1]Base pairs flanking introns, from Figs. 1 and 2. Bp1 is 12 bp upstream from the ATG, as in the original report of the cDNA sequence (Goyette et al. 1994).

Various doses of methotrexate (a drug used in treatment of cancer and arthritis, possibly other diseases) were added to colon carcinoma lines in culture. Much lower doses of methotrexate are needed to kill the lines that carry the 677C→T mutation in MTHFR, compared to lines that do not carry this mutation. The IC50 (concentration needed to kill half the cells) is approximately 20 nM for lines with the mutation and approximately 150 nM for lines without the mutation. To extrapolate to the human condition, patients with this MTHFR mutation might require lower doses of methotrexate for therapy, or might be subject to methotrexate toxicity at high doses.

Table 7

| Summary of BMD analysis | | | |
|---|---|---|---|
| **Entire cohort: Values are means +/- SE** | | | |
| | Genotype 1/1 | Genotype 1/2 | Genotype 2/2 |
| Spinal Z score | -1.06 (0.193) | -1.25 (0.176) | -1.86 (0.238) |
| Femoral neck Z score | -0.69 (0.134) | -0.78 (0.126) | -0.87 (0.228) |
| Trochanter Z score | -0.60 (0.142) | -0.52 (0.134) | -1.15 (0.249) |
| Ward's triangle Z score | -0.67 (0.147) | -0.80 (0.139) | -0.96 (0.257) |
| Values for bone mineral density in a group of individuals who were examined for the MTHFR 677C→T mutation.<br>Genotype 1/1 = normal C/C<br>Genotype 1/2 = carriers C/T<br>Genotype 2/2 = homozygous mutant T/T | | | |

As seen on Table 7, the lower the score, the lower the bone mineral density and therefore the higher the risk for osteoporosis. The results suggest that the homozygous mutant genotype (2/2) is asssociated with lower bone mineral density and therefore higher risk of osteoporosis.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. Use of an in-vitro method for identification of methylenetetrahydrofolate reductase (MTHFR) deficiency in a DNA sample or in a RNA sample obtained from a patient with MTHFR deficiency, said method comprising the steps of:

    a) amplifying the DNA sample or reverse-transcripting the RNA sample into a DNA and amplifying said DNA;

and

b) analyzing the amplified DNA of step a) to determine at least one sequence abnormality with respect to a human MTHFR encoded by a nucleotide sequence as set forth in SEQ ID NO. 1 or coding for an amino acid sequence as set forth in SEQ ID NO. 2, said sequence abnormality being indicative of MTHFR deficiency, for the diagnosis of osteoporosis in said patient.

**2.** The use according to claim 1, wherein said sequence abnormality comprises a mutation selected from a group consisting of 167G→A, 482G→A, 559C→T, 677C→T, 692C→T, 764C→T, 792+1G→A, 985C→T, 1015C→T, 1081C→T, 1298A→C and 1317T→C.

**3.** Use of a methylenetetrahydrofolate reductase (MTHFR) protein encoded by a nucleotide sequence as set forth in SEQ ID NO. 1 or having an amino acid sequence as set forth in SEQ ID NO. 2 for the manufacture of a medicament for the treatment of osteoporosis in a patient wherein said osteoporosis is associated with methylenetetrahydrofolate reductase (MTHFR) deficiency.

**4.** Use of a recombinant vector for expression of methylenetetrahydrofolate reductase (MTHFR) under the control of a suitable promoter, the MTHFR being encoded by a nucleotide sequence as set forth in SEQ ID NO. 1 or having an amino acid sequence as set forth in SEQ ID NO. 2, for the manufacture of a medicament for the treatment of osteoporosis in a patient wherein said osteoporosis is associated with MTHFR deficiency.

## Patentansprüche

**1.** Verwendung eines In-vitro-Verfahrens zur Identifizierung von Methylentetrahydrofolat-Reductase- (MTHFR) -Mangel in einer DNA-Probe oder in einer RNA-Probe, die von einem Patienten mit MTHFR-Mangel erhalten wurde, wobei das Verfahren die Schritte enthält:

a) Amplifizieren der DNA-Probe oder reverses Transkribieren der RNA-Probe in eine DNA und Amplifizieren der DNA; und

b) Analysieren der amplifizierten DNA aus Schritt a), um mindestens eine Sequenzanomalie hinsichtlich eines humanen MTHFR zu bestimmen, die von der Nukleotidsequenz kodiert wird, die wie in SEQ ID Nr. 1 dargelegt ist, oder die für eine Aminosäuresequenz kodiert, die wie in SEQ ID Nr. 2 dargelegt ist, wobei die Sequenzanomalie indikativ für MTHFR-Mangel ist, zur Diagnose von Osteoporose in dem Patienten.

**2.** Verwendung nach Anspruch 1, wobei die Sequenzanomalie eine Mutation umfasst, die ausgewählt ist aus einer Gruppe bestehend aus 167G→A, 482G→A, 559C→T, 677C→T, 692C→T, 764C→T, 792+1G→A, 985C→T, 1015C→T, 1081C→T, 1298A→C und 1317T→C.

**3.** Verwendung eines Methylentetrahydrofolat-Reductase- (MTHFR) -Proteins, das von einer Nukleotidsequenz kodiert wird, die wie in SEQ ID Nr. 1 dargelegt ist, oder eine Aminosäuresequenz besitzt, die wie in SEQ ID Nr. 2 dargelegt ist, zur Herstellung eines Arzneimittels zur Behandlung von Osteoporose in einem Patienten, wobei die Osteoporose mit einem Methylentetrahydrofolat-Reductase- (MTHFR) -Mangel verbunden ist.

**4.** Verwendung eines rekombinanten Vektors zur Expression von Methylentetrahydrofolat-Reductase (MTHFR) unter der Kontrolle eines geeigneten Promotors, wobei die MTHFR von einer Nukleotidsequenz kodiert wird, die wie in SEQ ID Nr. 1 dargelegt ist, oder eine Aminosäuresequenz besitzt, die wie in SEQ ID Nr. 2 dargelegt ist, zur Herstellung eines Arzneimittels zur Behandlung von Osteoporose in einem Patienten, wobei die Osteoporose mit MTHFR-Mangel verbunden ist.

## Revendications

**1.** Utilisation d'une méthode in vitro pour l'identification de la déficience en méthylènetétrahydrofolate réductase (MTHFR) dans un échantillon d'ADN ou un échantillon d'ARN obtenu d'un patient ayant une déficience en MTHFR, ladite méthode comprenant les étapes de :

a) l'amplification de l'échantillon d'ADN ou la transcription inverse de l'échantillon d'ARN en ADN et l'amplification dudit ADN ; et
b) l'analyse de l'ADN amplifié à l'étape a) pour déterminer au moins une anormalité de séquence par rapport au MTHFR humain codé par une séquence de nucléotides telle que décrite à SEQ ID N° 1 ou codant pour une séquence d'acide aminé telle que décrite dans SEQ ID N° 2, ladite anormalité de séquence étant indicative de la déficience en MTHFR, pour le diagnostic de l'ostéoporose dans ledit patient.

**2.** Utilisation selon la revendication 1, dans laquelle ladite anormalité de séquence comprend une mutation sélectionnée dans un groupe consistant en 167G→A, 482G→A, 559C→T, 677C→T, 692C→T, 764C→T, 792+1G→A, 985C→T, 1015C→T, 1081C→T, 1298A→C et 1317T→C.

**3.** Utilisation de la protéine de méthylènetétrahydrofolate réductase (MTHFR) codée par une séquence de nucléotides selon la SEQ ID N°1 ou ayant une séquence acide aminé selon la SEQ ID N° 2 pour la fabrication d'un médicament pour le traitement de l'ostéoporose dans un patient dans lequel ladite ostéoporose est associée avec la déficience en méthylènetétrahydrofolaté réductase (MTHFR).

**4.** Utilisation d'un vecteur recombinant pour l'expression de la méthylènetétrahydrofolate réductase (MTHFR) sous le contrôle d'un promoteur convenable, le MTHFR étant codé par une séquence de nucléotides selon la SEQ ID N° 1 ou ayant une séquence d'acide aminé selon la SEQ ID N° 2 pour la fabrication d'un médicament pour le traitement de l'ostéoporose dans un patient dans lequel ladite ostéoporose est associée avec la déficience en MTHFR.

```
         10          20          30          40          50
AAT TCC GGA GCC ATG GTG AAC GAA GCC AGA GGA AAC AGC AGC CTC AAC CCC
TTA AGG CCT CGG TAC CAC TTG CTT CGG TCT CCT TTG TCG TCG GAG TTG GGG
Asn Ser Gly Ala Met Val Asn Glu Ala Arg Gly Asn Ser Ser Leu Asn Pro›

        60          70          80          90         100
TGC TTG GAG GGC AGT GCC AGC AGT GGC AGT GAG AGC TCC AAA GAT AGT TCG
ACG AAC CTC CCG TCA CGG TCG TCA CCG TCA CTC TCG AGG TTT CTA TCA AGC
Cys Leu Glu Gly Ser Ala Ser Ser Gly Ser Glu Ser Ser Lys Asp Ser Ser›

       110         120         130         140         150
AGA TGT TCC ACC CCG GGC CTG GAC CCT GAG CGG CAT GAG AGA CTC CGG GAG
TCT ACA AGG TGG GGC CCG GAC CTG GGA CTC GCC GTA CTC TCT GAG GCC CTC
Arg Cys Ser Thr Pro Gly Leu Asp Pro Glu Arg His Glu Arg Leu Arg Glu›

      160         170         180         190         200
AAG ATG AGG CGG CGA TTG GAA TCT GGT GAC AAG TGG TTC TCC CTG GAA TTC
TTC TAC TCC GCC GCT AAC CTT AGA CCA CTG TTC ACC AAG AGG GAC CTT AAG
Lys Met Arg Arg Arg Leu Glu Ser Gly Asp Lys Trp Phe Ser Leu Glu Phe›

     210         220         230         240         250
TTC CCT CCT CGA ACT GCT GAG GGA GCT GTC AAT CTC ATC TCA AGG TTT GAC
AAG GGA GGA GCT TGA CGA CTC CCT CGA CAG TTA GAG TAG AGT TCC AAA CTG
Phe Pro Pro Arg Thr Ala Glu Gly Ala Val Asn Leu Ile Ser Arg Phe Asp›

    260         270         280         290         300
CGG ATG GCA GCA GGT GGC CCC CTC TAC ATA GAC GTG ACC TGG CAC CCA GCA
GCC TAC CGT CGT CCA CCG GGG GAG ATG TAT CTG CAC TGG ACC GTG GGT CGT
Arg Met Ala Ala Gly Gly Pro Leu Tyr Ile Asp Val Thr Trp His Pro Ala›

   310         320         330         340         350
GGT GAC CCT GGC TCA GAC AAG GAG ACC TCC TCC ATG ATG ATC GCC AGC ACC
CCA CTG GGA CCG AGT CTG TTC CTC TGG AGG AGG TAC TAC TAG CGG TCG TGG
Gly Asp Pro Gly Ser Asp Lys Glu Thr Ser Ser Met Met Ile Ala Ser Thr›
```

Fig. 1A

```
360        370        380        390        400
 *    *    *    *    *    *    *    *    *    *
GCC GTG AAC TAC TGT GGC CTG GAG ACC ATC CTG CAC ATG ACC TGC TGC CGT
CGG CAC TTG ATG ACA CCG GAC CTC TGG TAG GAC GTG TAC TGG ACG ACG GCA
Ala Val Asn Tyr Cys Gly Leu Glu Thr Ile Leu His Met Thr Cys Cys Arg>

410        420        430        440        450
 *    *    *    *    *    *    *    *    *    *
CAG CGC CTG GAG GAG ATC ACG GGC CAT CTG CAC AAA GCT AAG CAG CTG GGC
GTC GCG GAC CTC CTC TAG TGC CCG GTA GAC GTG TTT CGA TTC GTC GAC CCG
Gln Arg Leu Glu Glu Ile Thr Gly His Leu His Lys Ala Lys Gln Leu Gly>

460        470        480        490        500        510
 *    *    *    *    *    *    *    *    *    *    *
CTG AAG AAC ATC ATG GCG CTG CGG GGA GAC CCA ATA GGT GAC CAG TGG GAA
GAC TTC TTG TAG TAC CGC GAC GCC CCT CTG GGT TAT CCA CTG GTC ACC CTT
Leu Lys Asn Ile Met Ala Leu Arg Gly Asp Pro Ile Gly Asp Gln Trp Glu>

       520        530        540        550        560
 *    *    *    *    *    *    *    *    *    *
GAG GAG GAG GGA GGC TTC AAC TAC GCA GTG GAC CTG GTG AAG CAC ATC CGA
CTC CTC CTC CCT CCG AAG TTG ATG CGT CAC CTG GAC CAC TTC GTG TAG GCT
Glu Glu Glu Gly Gly Phe Asn Tyr Ala Val Asp Leu Val Lys His Ile Arg>

       570        580        590        600        610
 *    *    *    *    *    *    *    *    *    *
AGT GAG TTT GGT GAC TAC TTT GAC ATC TGT GTG GCA GGT TAC CCC AAA GGC
TCA CTC AAA CCA CTG ATG AAA CTG TAG ACA CAC CGT CCA ATG GGG TTT CCG
Ser Glu Phe Gly Asp Tyr Phe Asp Ile Cys Val Ala Gly Tyr Pro Lys Gly>

       620        630        640        650        660
 *    *    *    *    *    *    *    *    *    *
CAC CCC GAA GCA GGG AGC TTT GAG GCT GAC CTG AAG CAC TTG AAG GAG AAG
GTG GGG CTT CGT CCC TCG AAA CTC CGA CTG GAC TTC GTG AAC TTC CTC TTC
His Pro Glu Ala Gly Ser Phe Glu Ala Asp Leu Lys His Leu Lys Glu Lys>

       670        680        690        700        710
 *    *    *    *    *    *    *    *    *    *
GTG TCT GCG GGA GCC GAT TTC ATC ATC ACG CAG CTT TTC TTT GAG GCT GAC
CAC AGA CGC CCT CGG CTA AAG TAG TAG TGC GTC GAA AAG AAA CTC CGA CTG
Val Ser Ala Gly Ala Asp Phe Ile Ile Thr Gln Leu Phe Phe Glu Ala Asp>
```

Fig. 1B

```
     720        730        740        750        760
ACA TTC TTC CGC TTT GTG AAG GCA TGC ACC GAC ATG GGC ATC ACT TGC CCC
TGT AAG AAG GCG AAA CAC TTC CGT ACG TGG CTG TAC CCG TAG TGA ACG GGG
Thr Phe Phe Arg Phe Val Lys Ala Cys Thr Asp Met Gly Ile Thr Cys Pro>

    770        780        790        800        810
ATC GTC CCC GGG ATC TTT CCC ATC CAG GGC TAC CAC TCC CTT CGG CAG CTT
TAG CAG GGG CCC TAG AAA GGG TAG GTC CCG ATG GTG AGG GAA GCC GTC GAA
Ile Val Pro Gly Ile Phe Pro Ile Gln Gly Tyr His Ser Leu Arg Gln Leu>

  820        830        840        850        860
GTG AAG CTG TCC AAG CTG GAG GTG CCA CAG GAG ATC AAG GAC GTG ATT GAG
CAC TTC GAC AGG TTC GAC CTC CAC GGT GTC CTC TAG TTC CTG CAC TAA CTC
Val Lys Leu Ser Lys Leu Glu Val Pro Gln Glu Ile Lys Asp Val Ile Glu>

870        880        890        900        910
CCA ATC AAA GAC AAC GAT GCT GCC ATC CGC AAC TAT GGC ATC GAG CTG GCC
GGT TAG TTT CTG TTG CTA CGA CGG TAG GCG TTG ATA CCG TAG CTC GAC CGG
Pro Ile Lys Asp Asn Asp Ala Ala Ile Arg Asn Tyr Gly Ile Glu Leu Ala>

920        930        940        950        960
GTG AGC CTG TGC CAG GAG CTT CTG GCC AGT GGC TTG GTG CCA GGC CTC CAC
CAC TCG GAC ACG GTC CTC GAA GAC CGG TCA CCG AAC CAC GGT CCG GAG GTG
Val Ser Leu Cys Gln Glu Leu Leu Ala Ser Gly Leu Val Pro Gly Leu His>

970        980        990        1000        1010        1020
TTC TAC ACC CTC AAC CGC GAG ATG GCT ACC ACA GAG GTG CTG AAG CGC CTG
AAG ATG TGG GAG TTG GCG CTC TAC CGA TGG TGT CTC CAC GAC TTC GCG GAC
Phe Tyr Thr Leu Asn Arg Glu Met Ala Thr Thr Glu Val Leu Lys Arg Leu>

           1030        1040        1050        1060        1070
GGG ATG TGG ACT GAG GAC CCC AGG CGT CCC CTA CCC TGG GCT CTC AGT GCC
CCC TAC ACC TGA CTC CTG GGG TCC GCA GGG GAT GGG ACC CGA GAG TCA CGG
Gly Met Trp Thr Glu Asp Pro Arg Arg Pro Leu Pro Trp Ala Leu Ser Ala>
```

Fig. 1C

```
      1080      1090      1100      1110      1120
CAC CCC AAG CGC CGA GAG GAA GAT GTA CGT CCC ATC TTC TGG GCC TCC AGA
GTG GGG TTC GCG GCT CTC CTT CTA CAT GCA GGG TAG AAG ACC CGG AGG TCT
His Pro Lys Arg Arg Glu Glu Asp Val Arg Pro Ile Phe Trp Ala Ser Arg›

      1130      1140      1150      1160      1170
CCA AAG AGT TAC ATC TAC CGT ACC CAG GAG TGG GAC GAG TTC CCT AAC GGC
GGT TTC TCA ATG TAG ATG GCA TGG GTC CTC ACC CTG CTC AAG GGA TTG CCG
Pro Lys Ser Tyr Ile Tyr Arg Thr Gln Glu Trp Asp Glu Phe Pro Asn Gly›

      1180      1190      1200      1210      1220
CGC TGG GGC AAT TCC TCT TCC CCT GCC TTT GGG GAG CTG AAG GAC TAC TAC
GCG ACC CCG TTA AGG AGA AGG GGA CGG AAA CCC CTC GAC TTC CTG ATG ATG
Arg Trp Gly Asn Ser Ser Ser Pro Ala Phe Gly Glu Leu Lys Asp Tyr Tyr›

      1230      1240      1250      1260      1270
CTC TTC TAC CTG AAG AGC AAG TCC CCC AAG GAG GAG CTG CTG AAG ATG TGG
GAG AAG ATG GAC TTC TCG TTC AGG GGG TTC CTC CTC GAC GAC TTC TAC ACC
Leu Phe Tyr Leu Lys Ser Lys Ser Pro Lys Glu Glu Leu Leu Lys Met Trp›

      1280      1290      1300      1310      1320
GGG GAG GAG CTG ACC AGT GAA GCA AGT GTC TTT GAA GTC TTT GTT CTT TAC
CCC CTC CTC GAC TGG TCA CTT CGT TCA CAG AAA CTT CAG AAA CAA GAA ATG
Gly Glu Glu Leu Thr Ser Glu Ala Ser Val Phe Glu Val Phe Val Leu Tyr›

      1330      1340      1350      1360      1370
CTC TCG GGA GAA CCA AAC CGG AAT GGT CAC AAA GTG ACT TGC CTG CCC TGG
GAG AGC CCT CTT GGT TTG GCC TTA CCA GTG TTT CAC TGA ACG GAC GGG ACC
Leu Ser Gly Glu Pro Asn Arg Asn Gly His Lys Val Thr Cys Leu Pro Trp›

      1380      1390      1400      1410      1420
AAC GAT GAG CCC CTG GCG GCT GAG ACC AGC CTG CTG AAG GAG GAG CTG CTG
TTG CTA CTC GGG GAC CGC CGA CTC TGG TCG GAC GAC TTC CTC CTC GAC GAC
Asn Asp Glu Pro Leu Ala Ala Glu Thr Ser Leu Leu Lys Glu Glu Leu Leu›
```

Fig. 1D

```
1430        1440        1450        1460        1470
CGG GTG AAC CGC CAG GGC ATC CTC ACC ATC AAC TCA CAG CCC AAC ATC AAC
GCC CAC TTG GCG GTC CCG TAG GAG TGG TAG TTG AGT GTC GGG TTG TAG TTG
Arg Val Asn Arg Gln Gly Ile Leu Thr Ile Asn Ser Gln Pro Asn Ile Asn>

1480        1490        1500        1510        1520        1530
GGG AAG CCG TCC TCC GAC CCC ATC GTG GGC TGG GGC CCC AGC GGG GGC TAT
CCC TTC GGC AGG AGG CTG GGG TAG CAC CCG ACC CCG GGG TCG CCC CCG ATA
Gly Lys Pro Ser Ser Asp Pro Ile Val Gly Trp Gly Pro Ser Gly Gly Tyr>

      1540        1550        1560        1570        1580
GTC TTC CAG AAG GCC TAC TTA GAG TTT TTC ACT TCC CGC GAG ACA GCG GAA
CAG AAG GTC TTC CGG ATG AAT CTC AAA AAG TGA AGG GCG CTC TGT CGC CTT
Val Phe Gln Lys Ala Tyr Leu Glu Phe Phe Thr Ser Arg Glu Thr Ala Glu>

     1590        1600        1610        1620        1630
GCA CTT CTG CAA GTG CTG AAG AAG TAC GAG CTC CGG GTT AAT TAC CAC CTT
CGT GAA GAC GTT CAC GAC TTC TTC ATG CTC GAG GCC CAA TTA ATG GTG GAA
Ala Leu Leu Gln Val Leu Lys Lys Tyr Glu Leu Arg Val Asn Tyr His Leu>

    1640        1650        1660        1670        1680
GTC AAT GTG AAG GGT GAA AAC ATC ACC AAT GCC CCT GAA CTG CAG CCG AAT
CAG TTA CAC TTC CCA CTT TTG TAG TGG TTA CGG GGA CTT GAC GTC GGC TTA
Val Asn Val Lys Gly Glu Asn Ile Thr Asn Ala Pro Glu Leu Gln Pro Asn>

   1690        1700        1710        1720        1730
GCT GTC ACT TGG GGC ATC TTC CCT GGG CGA GAG ATC ATC CAG CCC ACC GTA
CGA CAG TGA ACC CCG TAG AAG GGA CCC GCT CTC TAG TAG GTC GGG TGG CAT
Ala Val Thr Trp Gly Ile Phe Pro Gly Arg Glu Ile Ile Gln Pro Thr Val>

  1740        1750        1760        1770        1780
GTG GAT CCC GTC AGC TTC ATG TTC TGG AAG GAC GAG GCC TTT GCC CTG TGG
CAC CTA GGG CAG TCG AAG TAC AAG ACC TTC CTG CTC CGG AAA CGG GAC ACC
Val Asp Pro Val Ser Phe Met Phe Trp Lys Asp Glu Ala Phe Ala Leu Trp>
```

Fig. 1E

```
    1790        1800        1810        1820        1830
ATT GAG CGG TGG GGA AAG CTG TAT GAG GAG GAG TCC CCG TCC CGC ACC ATC
TAA CTC GCC ACC CCT TTC GAC ATA CTC CTC CTC AGG GGC AGG GCG TGG TAG
Ile Glu Arg Trp Gly Lys Leu Tyr Glu Glu Glu Ser Pro Ser Arg Thr Ile>
```

```
   1840        1850        1860        1870        1880
ATC CAG TAC ATC CAC GAC AAC TAC TTC CTG GTC AAC CTG GTG GAC AAT GAC
TAG GTC ATG TAG GTG CTG TTG ATG AAG GAC CAG TTG GAC CAC CTG TTA CTG
Ile Gln Tyr Ile His Asp Asn Tyr Phe Leu Val Asn Leu Val Asp Asn Asp>
```

```
1890        1900        1910        1920        1930
TTC CCA CTG GAC AAC TGC CTC TGG CAG GTG GTG GAA GAC ACA TTG GAG CTT
AAG GGT GAC CTG TTG ACG GAG ACC GTC CAC CAC CTT CTG TGT AAC CTC GAA
Phe Pro Leu Asp Asn Cys Leu Trp Gln Val Val Glu Asp Thr Leu Glu Leu>
```

```
1940        1950        1960        1970        1980        1990
CTC AAC AGG CCC ACC CAG AAT GCG AGA GAA ACG GAG GCT CCA TGACCCTGCG
GAG TTG TCC GGG TGG GTC TTA CGC TCT CTT TGC CTC CGA GGT ACTGGGACGC
Leu Asn Arg Pro Thr Gln Asn Ala Arg Glu Thr Glu Ala Pro>
```

```
      2000       2010       2020       2030       2040       2050
TCCTGACGCC CTGCGTTGGA GCCACTCCTG TCCCGCCTTC CTCCTCCACA GTGCTGCTTC
AGGACTGCGG GACGCAACCT CGGTGAGGAC AGGGCGGAAG GAGGAGGTGT CACGACGAAG
```

```
      2060       2070       2080       2090       2100       2110
TCTTGGGAAC TCCACTCTCC TTCGTGTCTC TCCCACCCCG GCCTCCACTC CCCCACCTGA
AGAACCCTTG AGGTGAGAGG AAGCACAGAG AGGGTGGGGC CGGAGGTGAG GGGGTGGACT
```

```
      2120       2130       2140       2150       2160       2170
CAATGGCAGC TAGACTGGAG TGAGGCTTCC AGGCTCTTCC TGGACCTGAG TCGGCCCCAC
GTTACCGTCG ATCTGACCTC ACTCCGAAGG TCCGAGAAGG ACCTGGACTC AGCCGGGGTG
```

```
      2180       2190       2200       2210       2220
ATGGGAACCT AGTACTCTCT GCTCTAAAAA AAAAAAAAAA AAAGGAATTC
TACCCTTGGA TCATGAGAGA CGAGATTTTT TTTTTTTTTT TTTCCTTAAG
```

Fig. 1F

```
AMVNE ARGNS SLNPC LEGSA SSGSE SSKDS SRCST PGLDP ERHER LREKM RRRLE S--GDKW FSLEF   mthfr
                                              ms ffHas qRdal nqsLa evqGqin vSfEF   ecometf
                                              ms ffHan qREal nqsLa evqGqin vSfEF   stymetf
                                                        ms iRdLy haraspf iSLEF   ysRADI
                                    100·
FPPRT AEGAV NLISR FDRMA AGGPL YIDVT WHPAG DPGSD KETSS MMIAS TAVNY CGLET ILHMT   mthfr
FPPRT sEmeq tLwns iDRls slkPk fvsVT y--ga nsGer drThs i-lkg ik-dr tGLEa apHIT   ecometf
FPPRT sEmeq tLwns iDRls slkPk fvsVT y--ga nsGer drThs v-lkg ik-er tGLEa apHIT   stymetf
FPPkT elGtr NLmeR mhRMt AldPL fltVT W--ga -gGtt aEktl t-lAS lAqqt lnipv cmHIT   ysRADI
                                *                                  *
CCRQR LEEIT GHLHK AKQLG LKNIM ALRGD -PIGDQ WEEEE GGFNY AVGLV KHIRS EFGDY FDICV   mthfr
Cidat pdElr tiard ywnnG irhIv ALRGD lPpGsg kpE-- ---mY AsdLV tllk- EvaD- FDIsV   ecometf
Cidat rdElr tiard ywnnG irhIv ALRGD lPpGsg kpE-- ---mY AadLV gllk- EvaD- FDIsV   stymetf
Ctnte kaild daLdr cynaG irNIl ALRGn lPIGvv Wlvsq snrll nmrLf>                 ysRADI
 200·
AGYPK GHPEA GSFEA DLKHL KEKVS AGADF IITQL FFEAD TFFRF VKACT DMGIT CPIVP GIFPI   mthfr
AaYPe vHPEA kSaqA DLlnL KrKVd AGAnr aITQf FFdve sylRF rdrCv saGId veliP GIlPv   ecometf
AaYPe vHPEA kSaqA DLlnL KrKVd AGAnr aITQf FFdve sylRF rdrCv saGId veliP GIlPv   stymetf
                                                300·
QGYHS LRQLV KLSKL EVPQE IKDVI EPIKD NDAAI RN-YGI ELAVS LCQEL LASGL VPGLH FYTLN   mthfr
snfkq akkfa dmtnv riPaw maqmf dgl-D dDAet RklvGa niAmd mvkiL sreG- VkdfH FYTLN   ecometf
snfkq akkfa dmtnv riPsw mslmf Egl-D nDAet RklvGa niAmd mvkiL sreG- VkdfH FYTLN   stymetf

R-EMAT TEVLK RLGMW TEDPR RPLPW ALSAH PKRRE EDVRP IFWAS RPKSY IYRTD EWDEF PNGRW   mthfr
RaEMsy a-ich tLGvr pgl>                                                        ecometf
RaEMsy a-ich tLGvr pgl>                                                        stymetf
     400·
GNSSS PAFGE LKDYY LFYLK SKSPK E  mthfr
```

Fig. 2

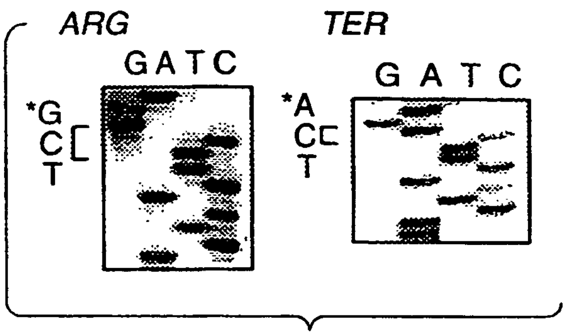


Fig. 3A

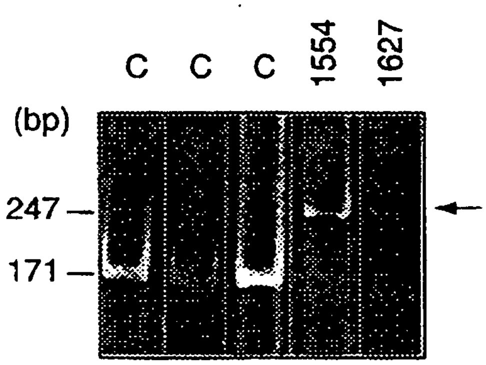


Fig. 3B

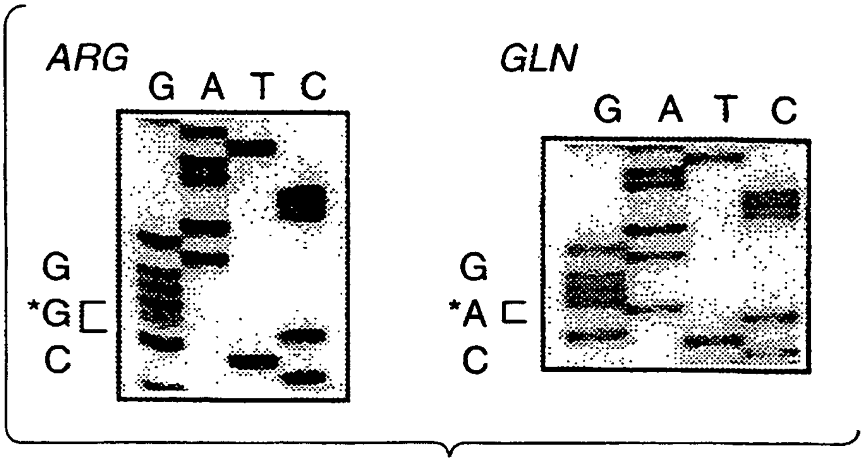


Fig. 4A

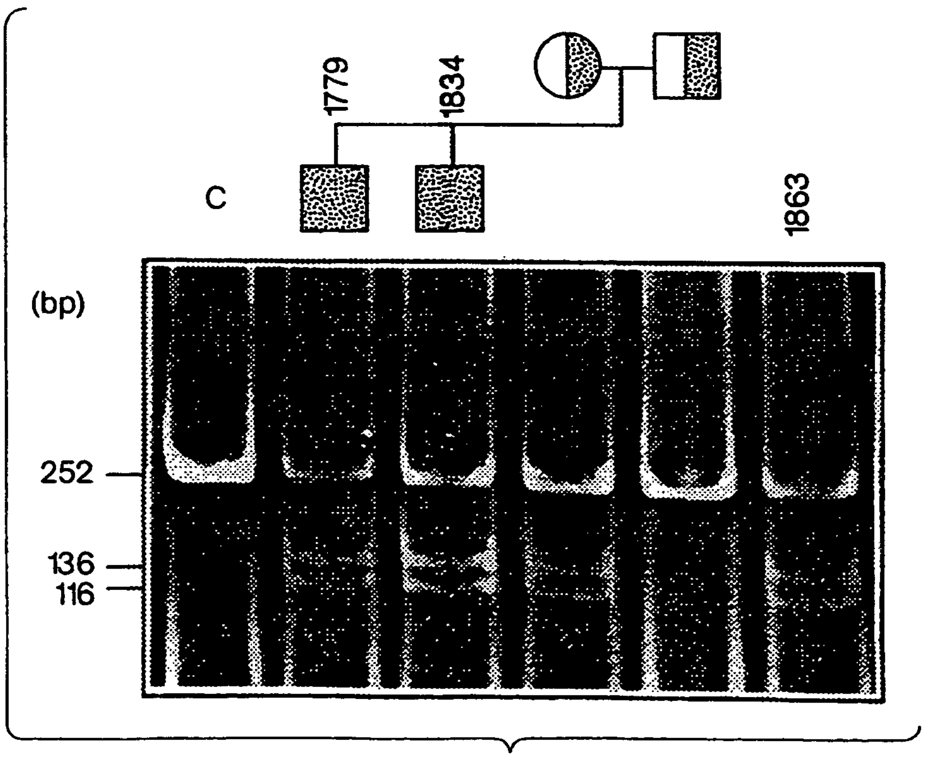


Fig. 4B

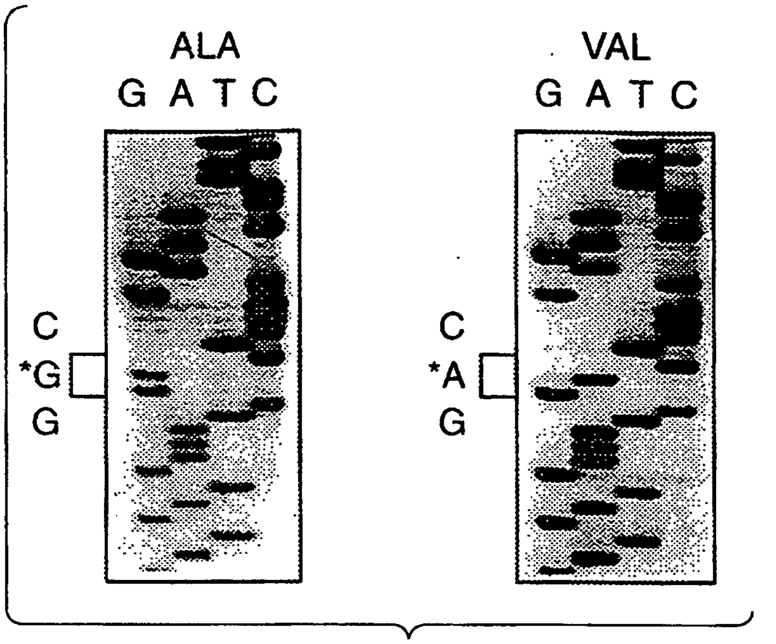


Fig. 5A

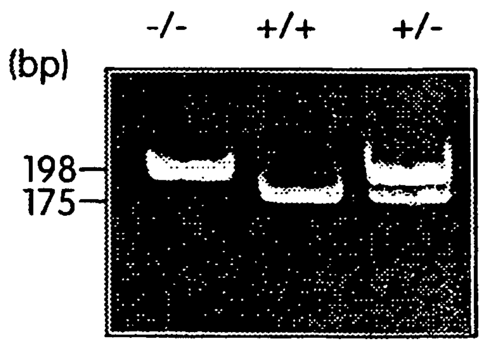


Fig. 5B

```
AAT TCC GGA GCC ATG GTG AAC GAA GCC AGA GGA AAC AGC AGC CTC AAC CCC TGC TTG GAG   60
                Met Val Asn Glu Ala Arg Gly Asn Ser Ser Leu Asn Pro Cys Leu Glu   16

GGC AGT GCC AGC AGT GGC AGT GAG AGC TCC AAA GAT AGT TCG AGA TGT TCC ACC CCG GGC  120
Gly Ser Ala Ser Ser Gly Ser Glu Ser Ser Lys Asp Ser Ser Arg Cys Ser Thr Pro Gly   36

CTG GAC CCT GAG CGG CAT GAG AGA CTC CGG GAG AAG ATG AGG CGG CGA TTG GAA TCT GGT  180
Leu Asp Pro Glu Arg His Glu Arg Leu Arg Glu Lys Met Arg Arg Arg Leu Glu Ser Gly   56

GAC AAG TGG TTC TCC CTG GAA TTC TTC CCT CCT CGA ACT GCT GAG GGA GCT GTC AAT CTC  240
Asp Lys Trp Phe Ser Leu Glu Phe Phe Pro Pro Arg Thr Ala Glu Gly Ala Val Asn Leu   76

ATC TCA AGG TTT GAC CGG ATG GCA GCA GGT GGC CCC CTC TAC ATA GAC GTG ACC TGG CAC  300
Ile Ser Arg Phe Asp Arg Met Ala Ala Gly Gly Pro Leu Tyr Ile Asp Val Thr Trp His   96

CCA GCA GGT GAC CCT GGC TCA GAC AAG GAG ACC TCC TCC ATG ATG ATC GCC AGC ACC GCC  360
Pro Ala Gly Asp Pro Gly Ser Asp Lys Glu Thr Ser Ser Met Met Ile Ala Ser Thr Ala  116

GTG AAC TAC TGT GGC CTG GAG ACC ATC CTG CAC ATG ACC TGC TGC CGT CAG CGC CTG GAG  420
Val Asn Tyr Cys Gly Leu Glu Thr Ile Leu His Met Thr Cys Cys Arg Gln Arg Leu Glu  136

GAG ATC ACG GGC CAT CTG CAC AAA GCT AAG CAG CTG GGC CTG AAG AAC ATC ATG GCG CTG  480
Glu Ile Thr Gly His Leu His Lys Ala Lys Gln Leu Gly Leu Lys Asn Ile Met Ala Leu  156

CGG GGA GAC CCA ATA GGT GAC CAG TGG GAA GAG GAG GAG GGA GGC TTC AAC TAC GCA GTG  540
Arg Gly Asp Pro Ile Gly Asp Gln Trp Glu Glu Glu Glu Gly Gly Phe Asn Tyr Ala Val  176

GAC CTG GTG AAG CAC ATC CGA AGT GAG TTT GGT GAC TAC TTT GAC ATC TGT GTG GCA GGT  600
Asp Leu Val Lys His Ile Arg Ser Glu Phe Gly Asp Tyr Phe Asp Ile Cys Val Ala Gly  196

TAC CCC AAA GGC CAC CCC GAA GCA GGG AGC TTT GAG GCT GAC CTG AAG CAC TTG AAG GAG  660
Tyr Pro Lys Gly His Pro Glu Ala Gly Ser Phe Glu Ala Asp Leu Lys His Leu Lys Glu  216

AAG GTG TCT GCG GGA GCC GAT TTC ATC ATC ACG CAG CTT TTC TTT GAG GCT GAC ACA TTC  720
Lys Val Ser Ala Gly Ala Asp Phe Ile Ile Thr Gln Leu Phe Phe Glu Ala Asp Thr Phe  236
```

Fig. 6A

TTC CGC TTT GTG AAG GCA TGC ACC GAC ATG GGC ATC ACT TGC CCC ATC GTC CCC GGG ATC 780
Phe Arg Phe Val Lys Ala Cys Thr Asp Met Gly Ile Thr Cys Pro Ile Val Pro Gly Ile 256

TTT CCC ATC CAG GGC TAC CAC TCC CTT CGG CAG CTT GTG AAG CTG TCC AAG CTG GAG GTG 840
Phe Pro Ile Gln Gly Tyr His Ser Leu Arg Gln Leu Val Lys Leu Ser Lys Leu Glu Val 276

CCA CAG GAG ATC AAG GAC GTG ATT GAG CCA ATC AAA GAC AAC GAT GCT GCC ATC CGC AAC 900
Pro Gln Glu Ile Lys Asp Val Ile Glu Pro Ile Lys Asp Asn Asp Ala Ala Ile Arg Asn 296

TAT GGC ATC GAG CTG GCC GTG AGC CTG TGC CAG GAG CTT CTG GCC AGT GGC TTG GTG CCA 960
Tyr Gly Ile Glu Leu Ala Val Ser Leu Cys Gln Glu Leu Leu Ala Ser Gly Leu Val Pro 316

GGC CTC CAC TTC TAC ACC CTC AAC CGC GAG ATG GCT ACC ACA GAG GTG CTG AAG CGC CTG 1020
Gly Leu His Phe Tyr Thr Leu Asn Arg Glu Met Ala Thr Thr Glu Val Leu Lys Arg Leu 336

GGG ATG TGG ACT GAG GAC CCC AGG CGT CCC CTA CCC TGG GCT CTC AGT GCC CAC CCC AAG 1080
Gly Met Trp Thr Glu Asp Pro Arg Arg Pro Leu Pro Trp Ala Leu Ser Ala His Pro Lys 356

CGC CGA GAG GAA GAT GTA CGT CCC ATC TTC TGG GCC TCC AGA CCA AAG AGT TAC ATC TAC 1140
Arg Arg Glu Glu Asp Val Arg Pro Ile Phe Trp Ala Ser Arg Pro Lys Ser Tyr Ile Tyr 376

CGT ACC CAG GAG TGG GAC GAG TTC CCT AAC GGC CGC TGG GGC AAT TCC TCT TCC CCT GCC 1200
Arg Thr Gln Glu Trp Asp Glu Phe Pro Asn Gly Arg Trp Gly Asn Ser Ser Ser Pro Ala 396

TTT GGG GAG CTG AAG GAC TAC TAC CTC TTC TAC CTG AAG AGC AAG TCC CCC AAG GAG GAG 1260
Phe Gly Glu Leu Lys Asp Tyr Tyr Leu Phe Tyr Leu Lys Ser Lys Ser Pro Lys Glu Glu 416

CTG CTG AAG ATG TGG GGG GAG GAG CTG ACC AGT GAA GCA AGT GTC TTT GAA GTC TTT GTT 1320
Leu Leu Lys Met Trp Gly Glu Glu Leu Thr Ser Glu Ala Ser Val Phe Glu Val Phe Val 436

CTT TAC CTC TCG GGA GAA CCA AAC CGG AAT GGT CAC AAA GTG ACT TGC CTG CCC TGG AAC 1380
Leu Tyr Leu Ser Gly Glu Pro Asn Arg Asn Gly His Lys Val Thr Cys Leu Pro Trp Asn 456

GAT GAG CCC CTG GCG GCT GAG ACC AGC CTG CTG AAG GAG GAG CTG CTG CGG GTG AAC CGC 1440
Asp Glu Pro Leu Ala Ala Glu Thr Ser Leu Leu Lys Glu Glu Leu Leu Arg Val Asn Arg 476

Fig. 6B

CAG GGC ATC CTC ACC ATC AAC TCA CAG CCC AAC ATC AAC GGG AAG CCG TCC TCC GAC CCC 1500
Gln Gly Ile Leu Thr Ile Asn Ser Gln Pro Asn Ile Asn Gly Lys Pro Ser Ser Asp Pro 496

ATC GTG GGC TGG GGC CCC AGC GGG GGC TAT GTC TTC CAG AAG GCC TAC TTA GAG TTT TTC 1560
Ile Val Gly Trp Gly Pro Ser Gly Gly Tyr Val Phe Gln Lys Ala Tyr Leu Glu Phe Phe 516

ACT TCC CGC GAG ACA GCG GAA GCA CTT CTG CAA GTG CTG AAG AAG TAC GAG CTC CGG GTT 1620
Thr Ser Arg Glu Thr Ala Glu Ala Leu Leu Gln Val Leu Lys Lys Tyr Glu Leu Arg Val 536

AAT TAC CAC CTT GTC AAT GTG AAG GGT GAA AAC ATC ACC AAT GCC CCT GAA CTG CAG CCG 1680
Asn Tyr His Leu Val Asn Val Lys Gly Glu Asn Ile Thr Asn Ala Pro Glu Leu Gln Pro 556

AAT GCT GTC ACT TGG GGC ATC TTC CCT GGG CGA GAG ATC ATC CAG CCC ACC GTA GTG GAT 1740
Asn Ala Val Thr Trp Gly Ile Phe Pro Gly Arg Glu Ile Ile Gln Pro Thr Val Val Asp 576

CCC GTC AGC TTC ATG TTC TGG AAG GAC GAG GCC TTT GCC CTG TGG ATT GAG CGG TGG GGA 1800
Pro Val Ser Phe Met Phe Trp Lys Asp Glu Ala Phe Ala Leu Trp Ile Glu Arg Trp Gly 596

AAG CTG TAT GAG GAG GAG TCC CCG TCC CGC ACC ATC ATC CAG TAC ATC CAC GAC AAC TAC 1860
Lys Leu Tyr Glu Glu Glu Ser Pro Ser Arg Thr Ile Ile Gln Tyr Ile His Asp Asn Tyr 616

TTC CTG GTC AAC CTG GTG GAC AAT GAC TTC CCA CTG GAC AAC TGC CTC TGG CAG GTG GTG 1920
Phe Leu Val Asn Leu Val Asp Asn Asp Phe Pro Leu Asp Asn Cys Leu Trp Gln Val Val 636

GAA GAC ACA TTG GAG CTT CTC AAC AGG CCC ACC CAG AAT GCG AGA GAA ACG GAG GCT CCA 1980
Glu Asp Thr Leu Glu Leu Leu Asn Arg Pro Thr Gln Asn Ala Arg Glu Thr Glu Ala Pro 656

TGA CCC TGC GTC CTG ACG CCC TGC GTT GGA GCC ACT CCT GTC CCG CCT TCC TCC TCC ACA 2040
End

GTG CTG CTT CTC TTG GGA ACT CCA CTC TCC TTC GTG TCT CTC CCA CCC CGG CCT CCA CTC 2100

CCC CAC CTG ACA ATG GCA GCT AGA CTG GAG TGA GGC TTC CAG GCT CTT CCT GGA CCT GAG 2160

TCG GCC CCA CAT GGG AAC CTA GTA CTC TCT GCT CTA AAA AAA AAA AAA AAA AAG GAA TT 2220

Fig. 6C

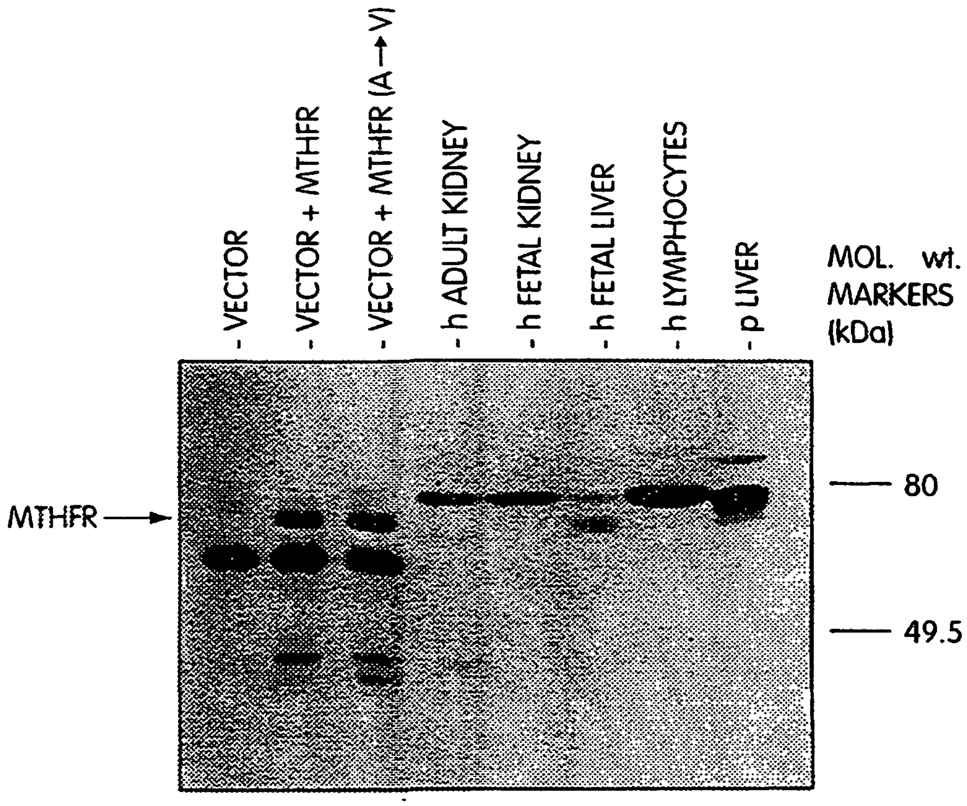


Fig. 7A

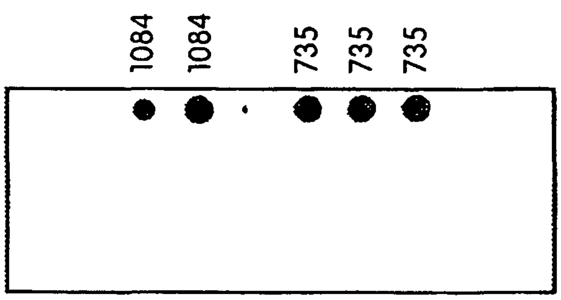


Fig. 10A

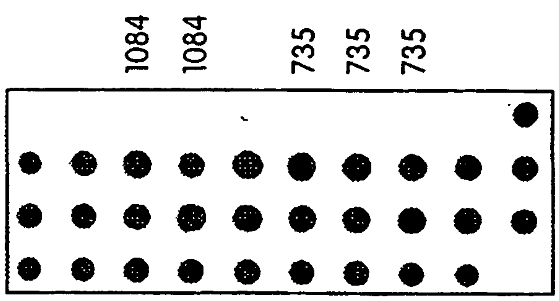


Fig. 10B

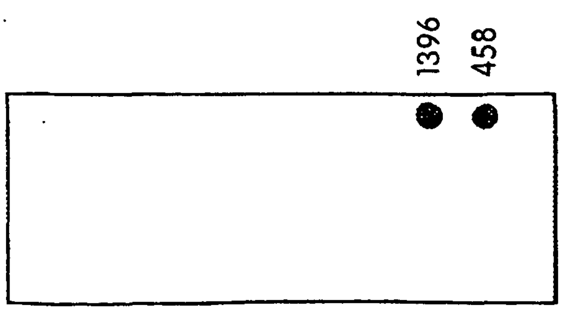


Fig. 10C

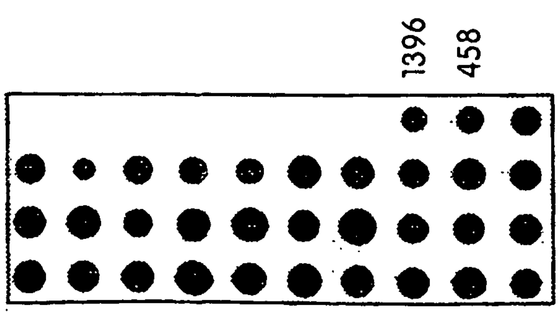


Fig. 10D

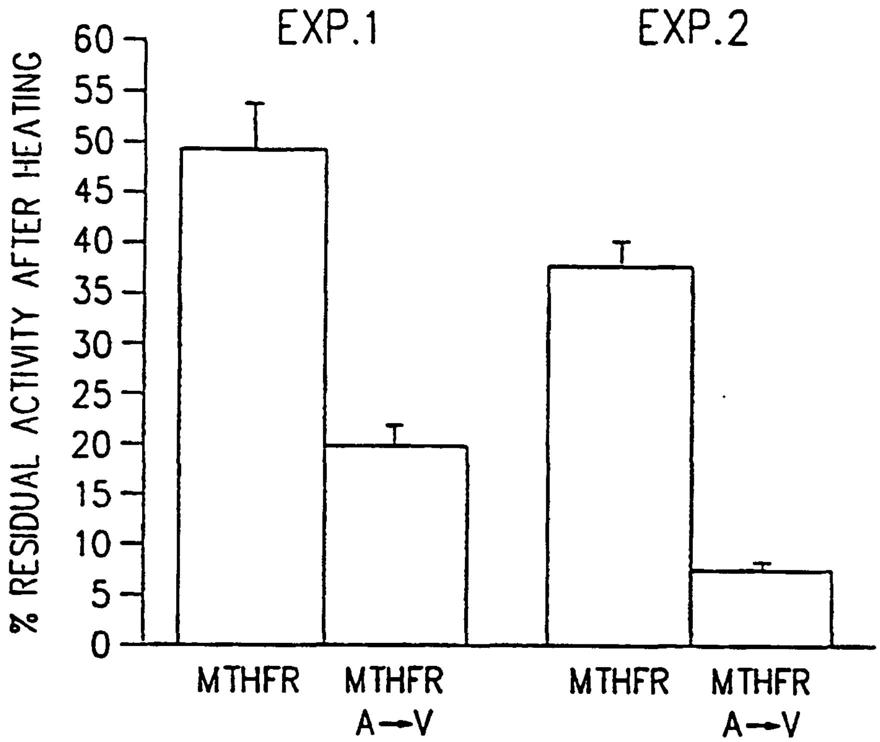


Fig. 7B

```
                   *
MTHFR:   KHLKEKVSAGADFIITQLFFEADTFFR
          |||  |·       |  |  ||·||||
DHFR:    GHLKLFVT----R-IMQD-FESDTFFP
```

Fig. 11

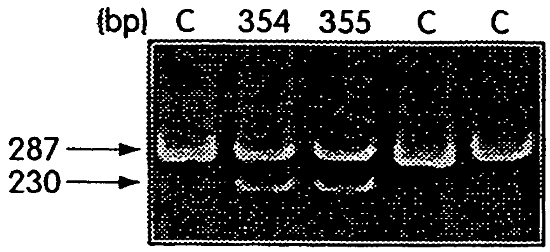
(bp)
C
354
355
C
C
287
230

Fig. 8A

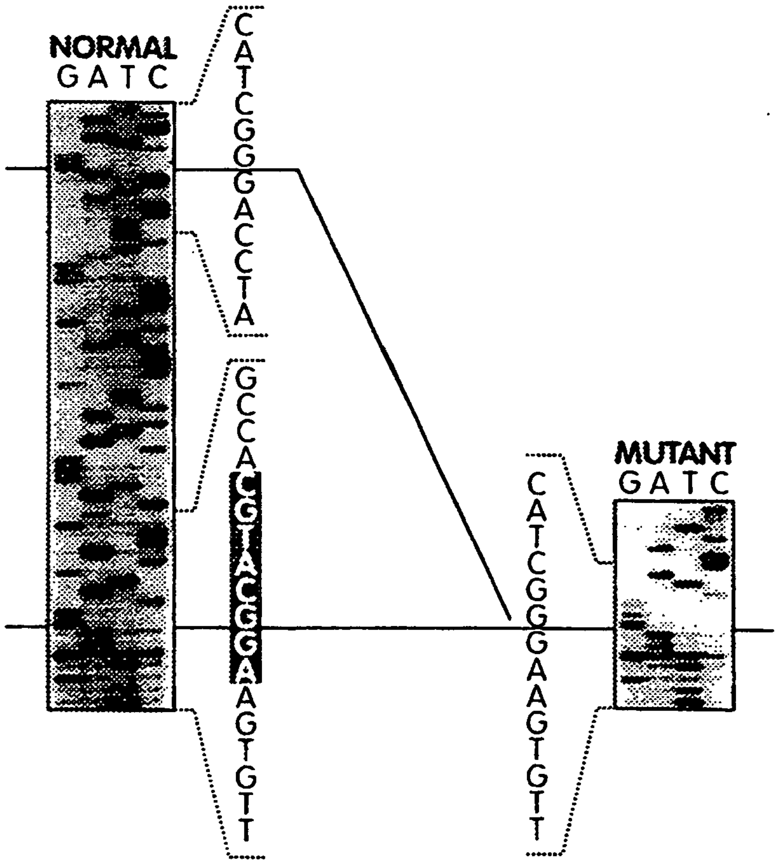
NORMAL
G A T C
C
A
T
C
G
G
G
A
C
C
T
A
G
C
C
A
C
G
T
A
C
G
G
A
A
G
T
G
T
T
MUTANT
G A T C
C
A
T
C
G
G
G
A
A
G
T
G
T
T

Fig. 8B

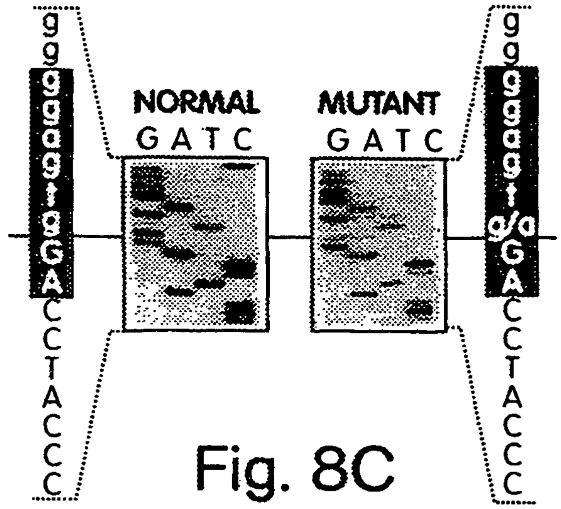
NORMAL
G A T C
MUTANT
G A T C
g g g g a g t g G A C C T A C C C
g g g g a g t g/a G A C C T A C C C

Fig. 8C

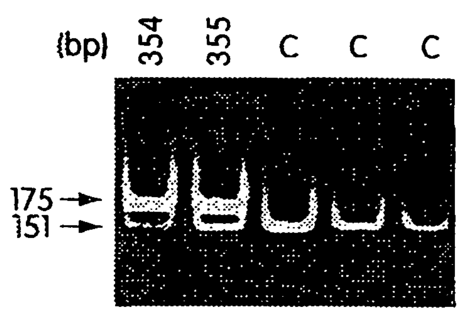
(bp)
354
355
C
C
C
175
151

Fig. 8D

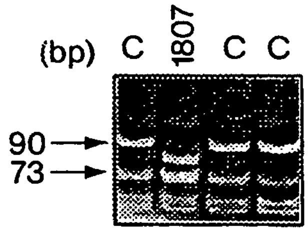


Fig. 9A

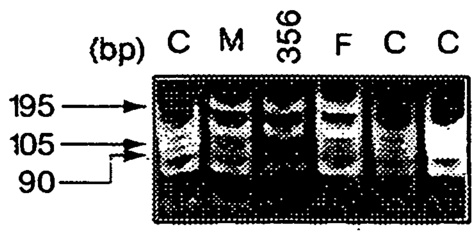


Fig. 9B

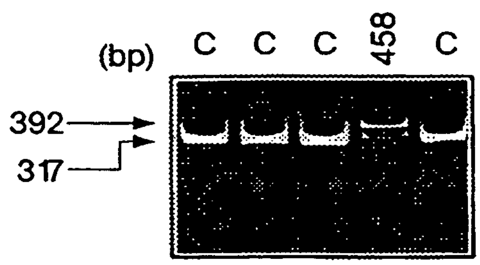


Fig. 9C

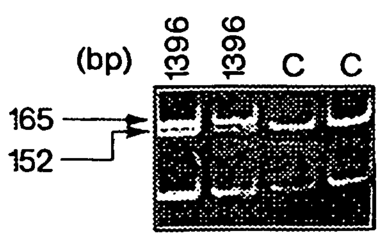


Fig. 9D

EXON 1: 246 bp (bp 3-248)

*

gggtgtggct gcctgccccc tgatgctccc tgccccaccc tgtgcagtag **GAACCCAGCC**
**ATGGTGAACG AAGCCAGAGG AAACAGCAGC CTCAACCCCT GCTTGGAGGG CAGTGCCAGC**
**AGTGGCAGTG AGAGCTCCAA AGATAGTTCG AGATGTTCCA CCCCGGGCCT GGACCCTGAG**
**CGGCATGAGA GACTCCGGGA GAAGATGAGG CGGCGATTGG AATCTGGTGA CAAGTGGTTC**
**TCCCTGGAAT TCTTCCCTCC TCGAACTGCT GAGGGAGCTG TCAATCTCAT CTCAAG**gtaa
actcatgcaa ggttaaggtg agaggcggga gtggtggtgc ctgggg

EXON 2: 239 bp (bp 249-487)

acggatgg tatttctcct ggaacctctc ttcagaaaca aaccccctacag **GTTTGACCGG**
**ATGGCAGCAG GTGGCCCCCT CTACATAGAC GTGACCTGGC ACCCAGCAGG TGACCCTGGC**
**TCAGACAAGG AGACCTCCTC CATGATGATC GCCAGCACCG CCGTGAACTA CTGTGGCCTG**
**GAGACCATCC TGCACATGAC CTGCTGCCGT CAGCGCCTGG AGGAGATCAC GGGCCATCTG**
**CACAAAGCTA AGCAGCTGGG CCTGAAGAAC ATCATGGCGC TGCGGGGAG**g tgtggagcca
gcactcccct acactctggg ttctggcttt cccggaggc

EXON 3: 111 bp (bp 488-598)

tctggaggtt gggtgagacc cagtgactat gacctccacc aaccctgcag **ACCCAATAGG**
**TGACCAGTGG GAAGAGGAGG AGGGAGGCTT CAACTACGCA GTGGACCTGG TGAAGCACAT**
**CCGAAGTGAG TTTGGTGACT ACTTTGACAT CTGTGTGGCA G**gtgagtggc tggatcatcc
tggtggcggg gatggagcta gggaggctga

EXON 4: 194 bp (bp 599-792)

ccttgaacag gtggaggcca gcctctcctg actgtcatcc ctattggcag **GTTACCCCAA**
**AGGCCACCCC GAAGCAGGGA GCTTTGAGGC TGACCTGAAG CACTTGAAGG AGAAGGTGTC**
**TGCGGGAGCC GATTTCATCA TCACGCAGCT TTTCTTTGAG GCTGACACAT TCTTCCGCTT**
**TGTGAAGGCA TGCACCGACA TGGGCATCAC TTGCCCCATC GTCCCCGGGA TCTTTCCCAT**
**CCAG**gtgagg ggcccaggag agcccataag ctccctccac cccactctca ccgc

EXON 5: 251 bp (bp 793-1043)

gctggccagc agccgccaca gcccctcatg tcttggacag **GGCTACCACT CCCTTCGGCA**
**GCTTGTGAAG CTGTCCAAGC TGGAGGTGCC ACAGGAGATC AAGGACGTGA TTGAGCCAAT**
**CAAAGACAAC GATGCTGCCA TCCGCAACTA TGGCATCGAG CTGGCCGTGA GCCTGTGCCA**
**GGAGCTTCTG GCCAGTGGCT TGGTGCCAGG CCTCCACTTC TACACCCTCA ACCGCGAGAT**
**GGCTACCACA GAGGTGCTGA AGCGCCTGGG GATGTGGACT GAGGACCCCA G**gtgagggca
gtggcccaga gatccccaga ggagggtcca agagcagccc c

EXON 6: 135 bp (bp 1044-1178)

tccctctagc caatcccttg tctcaattct ctgtccccat cctcacccag **GCGTCCCCTA**
**CCCTGGGCTC TCAGTGCCCA CCCCAAGCGC CGAGAGGAAG ATGTACGTCC CATCTTCTGG**
**GCCTCCAGAC CAAAGAGTTA CATCTACCGT ACCCAGGAGT GGGACGAGTT CCCTAACGGC**
**CGCTG**gtgag ggcctgcaga ccttccttgc aaatacatct ttgttcttgg gagcg

Fig. 12A

EXON 7: 181 bp (bp 1179-1359)

actgccctct gtcaggagtg tgccctgacc tctgggcacc cctctgccag **GGGCAATTCC TCTTCCCCTG CCTTTGGGGA GCTGAAGGAC TACTACCTCT TCTACCTGAA GAGCAAGTCC CCCAAGGAGG AGCTGCTGAA GATGTGGGGG GAGGAGCTGA CCAGTGAAGC AAGTGTCTTT GAAGTCTTTG TTCTTTACCT CTCGGGAGAA CCAAACCGGA ATGGTCACAA A**gtgagtgat gctggaagtg gggaccctgg ttcatcccct gcccctggcc t

EXON 8: 183 bp (bp 1360-1542)

cagggtgcca aacctgatgg tcgccccagc cagctcaccg tctctcccag **GTGACTTGCC TGCCCTGGAA CGATGAGCCC CTGGCGGCTG AGACCAGCCT GCTGAAGGAG GAGCTGCTGC GGGTGAACCG CCAGGGCATC CTCACCATCA ACTCACAGCC CAACATCAAC GGGAAGCCGT CCTCCGACCC CATCGTGGGC TGGGGCCCCA GCGGGGGCTA TGTCTTCCAG AAG**gtgtggt agggaggcac ggggtgcccc cctctcttga ccggcacccg tgg

EXON 9: 102 bp (bp 1543-1644)

gggcgtctgg cagggctggg gttggtgaca ggcacctgtc tctcccacag **GCCTACTTAG AGTTTTTCAC TTCCCGCGAG ACAGCGGAAG CACTTCTGCA AGTGCTGAAG AAGTACGAGC TCCGGGTTAA TTACCACCTT GTCAATGTGA AG**gtaggcca ggccccacgg ttcccacaga gtaccaggcc cttcgttgaa ca

EXON 10: 120 bp (bp 1645-1764)

actccagttg ttcttggccc aggtcttacc cccaccccac atcccctcag **GGTGAAAACA TCACCAATGC CCCTGAACTG CAGCCGAATG CTGTCACTTG GGGCATCTTC CCTGGGCGAG AGATCATCCA GCCCACCGTA GTGGATCCCG TCAGCTTCAT GTTCTGGAAG** gtaaaggagc gggggcaagc ttgccccgcc cacctggaaa accgtgggga

EXON 11: 219 bp (stop codon) (bp 1765-1983)
432 bp (end of cDNA) (bp 1765-2196)

ctctgtgtgt gtgtgcatgt gtgcgtgtgt gcgggggtat gtgtgtgtag **GACGAGGCCT TTGCCCTGTG GATTGAGCGG TGGGGAAAGC TGTATGAGGA GGAGTCCCCG TCCCGCACCA TCATCCAGTA CATCCACGAC AACTACTTCC TGGTCAACCT GGTGGACAAT GACTTCCCAC TGGACAACTG CCTCTGGCAG GTGGTGGAAG ACACATTGGA GCTTCTCAAC AGGCCCACCC AGAATGCGAG AGAAACGGAG GCTCCATGAC CCTGCGTCCT GACGCCCTGC GTTGGAGCCA CTCCTGTCCC GCCTTCCTCC TCCACAGTGC TGCTTCTCTT GGGAACTCCA CTCTCCTTCG TGTCTCTCCC ACCCCGGCCT CCACTCCCCC ACCTGACAAT GGCAGCTAGA CTGGAGTGAG GCTTCCAGGC TCTTCCTGGA CCTGAGTCGG CCCCACATGG GAACCTAGTA CTCTCTGCTC TA**gccaggag tctgtgctct tttggtgggg agcacttgct cctgcagagg ac

Fig. 12B

EXON 1: 243 bp (bp 3-245)

*

gggtttggta ccagccctat aataccccg gcccccaccc tctacagcag GAATCCAGCC
ATGGTGAACG AGGCCAGAGG AAGTGGCAGT CCCAACCCGC GATCTGAGGG CAGCAGCAGT
GGCAGCGAGA GTTCCAAGGA CAGTTCAAGA TGTTCCACCC CCAGCCTGGA CCCAGAGCGG
CACGAGAGAC TCCGGGAGAA GATGAGGCGC AGAATGGACT CTGGTGACAA GTGGTTCTCC
CTGGAGTTCT TCCCCCCTCG AACTGCTGAG GGAGCTGTTA ACCTCATCTC CAGgtgagta
gggaggttaa tccgcggggg tcggcaggct tcaggggagc gtg

EXON 2: 239 bp (bp 246-484)

gagctcccta tttaccccag gagcctactt aaggaggaaa tcccctacag GTTTGACCGG
ATGGCAGCAG GGGGCCCCCT CTTCGTAGAT GTTACCTGGC ACCCAGCTGG AGACCCTGGC
TCAGACAAGG AGACCTCCTC CATGATGATC GCCAGCACAG CAGTAAACTA CTGCGGCTTG
GAAACCATCC TGCATATGAC CTGCTGCCAG CAGCGCCCGG AGGAGATCAC AGGCCATCTG
CACAGAGCCA AGCAGCTGGG CCTGAAGAAC ATAATGGCGC TGAGGGGAGg tgtggcgcca
gcacccctcc tctttgggtt cttgctttcc tgaaggctt

EXON 3: 111 bp (bp 485-595)

tctggaggtc aggggacacc cagtgaccat gacctccagc aaccctgcag ACCCTGTAGG
TGACCACTGG GAAGCAGAGG AAGGAGGCTT CAGCTATGCC ACAGACCTGG TGAAGCACAT
CCGGACCGAG TTTGCTGACT ATTTTGACAT CTGTGTGGCA Ggtaagtgag gacagagaag
ggtcaggatg agaggatagc cagctagtct t

EXON 4: 194 bp (bp 596-789)

gcaggtaggt tgagaccagc cccccactc ttcttgtctc ctcctggtag GTTACCCCAG
AGGCCACCCC GATGCAGAGA GCTTCGAGGA TGACCTGAAG CATTTGAAGG AGAAGGTATC
TGCAGGCGCC GACTTCATTA TCACTCAGCT CTTCTTTGAG GCCAGCACCT TCTTCAGCTT
TGTGAAGGCC TGCACAGAGA TAGGCATCTC TTGCCCTATC CTGCCTGGGA TCTTCCCTAT
TCAGgtgagg ggcttgggag gacctgattc cctccgtcca gtgcatgcgg aagt

EXON 5: 251 bp (bp 790-1040)

cagtggagca taggccagag atgaccccat gccccttgtg tctctgacag GGCTACACTT
CCCTTCGGCA GCTTGTAAAA CTGTCCAAGC TGGAGGTGCC ACAGAAGATC AAGGATGTAA
TTGAGCCCAT CAAAGACAAC GATGCTGCCA TCCGCAACTA CGGCATTGAG CTGGCTGTAA
GGCTGTGCCG GGAGCTGCTG GACAGTGGCT TGGTGCCAGG CCTCCACTTC TATACCCTCA
ACCGCGAGGT GGCCACCATG GAGGTGCTAA AGCAACTGGG CATGTGGACC GAGGACCCCA
Ggtgagcggt ggaagctgga ggcataccca tgagtcagag tcgcgcaggt g

EXON 6: 135 bp (bp 1041-1175)

ctagctcagt ctacctaagc ccttgtcttt tccctcttcc ttccctccag GCGTCCCTTG
CCCTGGGCTC TCAGTGCGCA TCCCAAGCGC CGGGAGGAAG ATGTCCGTCC CATCTTCTGG
GCCTCCAGAC CAAAGAGCTA CATCTACCGC ACACAGGACT GGGATGAGTT TCCTAACGGC
CGCTGgtgag gagagaagcc agggggtgtt aggaattgct ggtgcctggg tggaa

Fig. 13A

EXON 7: 181 bp (bp 1176-1356)

aataggacaa gatttacaac aaagtgcctt gtcccttata ctcctgccag **GGGTAATTCT**
**TCCTCACCAG CCTTTGGGGA GCTGAAAGAC TACTACCTCT TCTACCTGAA AAGCAAGTCC**
**CCCAGGGAGG AGCTGCTGAA GATGTGGGGC GAGGAGCTCA CCAGCGAAGA GAGTGTCTTT**
**GAAGTCTTTG AACACTACCT CTCTGGAGAG CCGAATCGCC ATGGCTACAG A**gtgagtggg
gtgaggagga acggcccagc tttgtctcag ccttgg

EXON 8: 183 bp (bp 1357-1539)

cccagtccca gactcagtgc tgccctcgct cagcgcaccc tgccctgcag **GTAACCTGCC**
**TGCCCTGGAA CGATGAACCC CTGGCAGCGG AAACCAGCCT GATGAAGGAA GAGCTGCTCC**
**GCGTGAACAG GCTGGGCATC CTCACCATCA ACTCTCAGCC CAACATCAAC GCAAAACCAT**
**CCTCAGACCC TGTTGTGGGC TGGGGCCCCA GTGGGGGTTA TGTCTTCCAG AAG**gtatgct
aggatgcagt actctcgata tccccaggga ctgacacaga acc

EXON 9: 102 bp (bp 1540-1641)

gagaacttgg caagtagtgg ggttgacatg ttgggtgtat tctccctcag **GCCTACCTCG**
**AATTCTTCAC CTCCCGTGAA ACTGTGGAGG CGCTTCTGCA GGTGCTGAAG ACATACGAGC**
**TGCGGGTCAA CTACCACATC GTGGACGTGA AG**gtaagcca gctccctccg gcttagacgc
agcaaggctt gaaaacacct aca

EXON 10: 120 bp (bp 1642-1761)

agcagtggga ggttgcggtc accctgcctc agccctgcct ctgttctcag **GGAGAGAACA**
**TCACTAATGC CCCTGAGCTG CAGCCCAATG CCGTGACGTG GGGCATCTTC CCGGGTCGAG**
**AGATCATCCA GCCTACTGTG GTGGACCCCA TCAGCTTCAT GTTCTGGAAG** gtaagggagt
gggagggagt ggaggaccct ggctaccgtg agagcccag

EXON 11: 216 bp (stop codon) (bp 1762-1977)

ggaggtacca gccgtgctga ccctgctcgt gtgtctctgt tcacacgtag **GATGAGGCCT**
**TTGCCCTGTG GATCGAGCAG TGGGGCAAGC TATACGAGGA GGAGTCGCCA TCCCGCATGA**
**TCATCCAATA CATCCATGAC AACTATTTCC TGGTCAACCT GGTGGACAAC GAGTTCCCGC**
**TGGACAGCTG CCTGTGGCAG GTGGTGGAGG ACACGTTTGA GCTGCTCAAC AGGCATCCCA**
**CGGAGAGAGA GACACAGGCT CCATGA**gcct gcatctctca acaggcacac catggagaga
gagacacagg ctctgtgagc cgtgcatccc tcaacaggca caccacggag agagagacac
aggctccgtg agcctgcatc ccggtatctt cctcacctgg agcccctctc cctcatctct
ctacaca

Fig. 13B

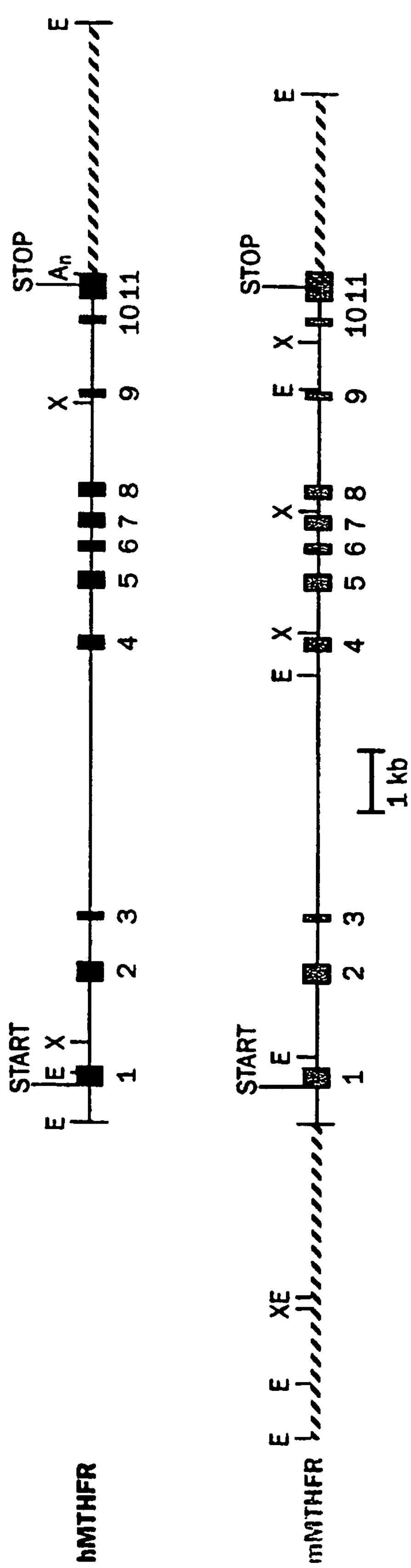


Fig. 14

hMTHFR MVNEARGNSSLNPCLEGSASSGSESSKDSSRCSTPGLDPERHERLREKMRRRLESGDKWF
mMTHFR □□□□□□□sg□ps□rs□□□-□□□□□□□□□□□□□□□□s□□□□□□□□□□□□□□□□mds□□□□□
bMTHFR ------------------------------------fhasqrda□nqsl-aevq-□qinv

hMTHFR SLEFFPPRTAEGAVNLISRFDRMAAGGPLYIDVTWHPAGDPGSDKETSSMMIASTAVNYC
mMTHFR □□□□□□□□□□□□□□□□□□□□□□□□□□□□□fv□□□□□□□□□□□□□□□□□□□□□□□□□□□□□
bMTHFR □f□□□□□□□s□meqt□wnsi□□lsslk□kfvs□□-yg□-ns□erdr□h□--□kgik-drt

hMTHFR GLETILHMTCCRQRLEEITGHLHKAKQLGLKNIMALRGDPIGDQWEEEEGGFNYAVDLVK
mMTHFR □□□□□□d□□□□q□□p□□□□□□□□r□□□□□□□□□□□□□□□□v□□h□□a□□□□□s□□t□□□□
bMTHFR □□□aap□l□□idatpd□lrtiardywnn□irh□v□□□□□lpp-gsgkp□m---□□s□□□t

hMTHFR HIRSEFGDYFDICVAGYPKGHPEAGSFEADLKHLKEKVSAGADFIITQLFFEADTFFRFV
mMTHFR □□□t□□a□□□□□□□□□□□r□□□d□e□□□d□□□□□□□□□□□□□□□□□□□□□□□□s□□□s□□
bMTHFR llk-□va□-□□□s□□a□□ev□□□□k□aq□□□ln□□r□□d□□□nra□□□f□□dvesyl□□r

hMTHFR KACTDMGITCPIVPGIFPIQGYHSLRQLVKLSKLEVPQEIKDVIEPIKDNDAAIRNYGIE
mMTHFR □□□□ei□□s□□□l□□□□□□□□□t□□□□□□□□□□□□□□□k□□□□□□□□□□□□□□□□□□□□□
bMTHFR dr□vsa□□dve□i□□□l□vsnfkqakkfadmtnvri□awmaqmfdgldDdaetrklv□an

hMTHFR LAVSLCQELLASGLVPGLHFYTLNREMATTEVLKRLGMWTEDPRRPLPWALSAHPKRREE
mMTHFR □□□x□□r□□□dS□□□□□□□□□□□□□□v□□m□□□□q□□□□□□□□□□□□□□□□□□□□□□□□□
bMTHFR i□mdmvk-i□sreg□kdf□□□□□□□aemsyaicht□□vr---------------------

hMTHFR DVRPIFWASRPKSYIYRTQEWDEFPNGRWGNSSSPAFGELKDYYLFYLKSKSPKEELLKM
mMTHFR □□□□□□□□□□□□□□□□□□□d□□□□□□□□□□□□□□□□□□□□□□□□□□□□□□□□□r□□□□□□
bMTHFR ------------------------------------------------------------

hMTHFR WGEELTSEASVFEVFVLYLSGEPNRNGHKVTCLPWNDEPLAAETSLLKEELLRVNRQGIL
mMTHFR □□□□□□□□e□□□□□□eh□□□□□□□□h□yr□□□□□□□□□□□□□□□□□m□□□□□□□□□l□□□
bMTHFR ------------------------------------------------------------

hMTHFR TINSQPNINGKPSSDPIVGWGPSGGYVFQKAYLEFFTSRETAEALLQVLKKYELRVNYHL
mMTHFR □□□□□□□□□a□□□□□□v□□□□□□□□□□□□□□□□□□□□□□□□v□□□□□□□□t□□□□□□□□i
bMTHFR ------------------------------------------------------------

hMTHFR VNVKGENITNAPELQPNAVTWGIFPGREIIQPTVVDPVSFMFWKDEAFALWIERWGKLYE
mMTHFR □d□□□□□□□□□□□□□□□□□□□□□□□□□□□□□□□□□i□□□□□□□□□□□□□□□q□□□□□□
bMTHFR ------------------------------------------------------------

hMTHFR EESPSRTIIQYIHDNYFLVNLVDNDFPLDNCLWQVVEDTLELLNRPTQNARETEAP
mMTHFR □□□□□□m□□□□□□□□□□□□□□□□□e□□□□s□□□□□□□□□f□□□□□h-pte□□□q□□
bMTHFR --------------------------------------------------------

Fig. 15